(19) Europäisches Patentamt
European Patent Office
Office européen des brevets

(11) Publication number: **0 258 175 B1**

(12) **EUROPEAN PATENT SPECIFICATION**

(45) Date of publication of patent specification: **01.04.92**

(51) Int. Cl.[5]: **C07D 471/04**, C07F 7/10, A61K 31/47, //(C07D471/04, 231:00,221:00)

(21) Application number: **87810387.8**

(22) Date of filing: **07.07.87**

The file contains technical information submitted after the application was filed and not included in this specification

(54) **5-hetero- or aryl-substituted-imidazo[2,1-a]isoquinolines.**

(30) Priority: **14.07.86 US 885115**
**11.02.87 US 13515**

(43) Date of publication of application:
**02.03.88 Bulletin 88/09**

(45) Publication of the grant of the patent:
**01.04.92 Bulletin 92/14**

(84) Designated Contracting States:
**AT BE CH DE ES FR GB GR IT LI LU NL SE**

(56) References cited:
**FR-A- 2 125 378**

(73) Proprietor: **SANDOZ AG**
**Lichtstrasse 35**
**CH-4002 Basel(CH)**
(84) Designated Contracting States:
**BE CH ES FR GB GR IT LI LU NL SE**

(73) Proprietor: **SANDOZ-PATENT-GMBH**
**Humboldtstrasse 3**
**W-7850 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

(72) Inventor: **Houlihan, William Joseph**
**15 Raynold Road**
**Mountain Lakes N.J.(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to certain 5-hetero-or aryl-substituted-imidazo[2,1-a]isoquinolines and to their use as platelet activating factor (PAF) receptor antagonists. The invention also relates to pharmaceutical compositions containing a select group of the afore-mentioned compounds as an active ingredient thereof. In addition, the invention relates to the use of a select group of said compounds as anti-tumor agents.

USP 3,887,566 discloses certain 2,3-dihydroimidazo-isoquinolines exhibiting analgesic, anti-inflammatory, anti-bacterial, anti-viral and cardiovascular properties. USP 4,100,165 discloses certain 5-hydroxy-2,3,5,6-tetrahydrofuran imidazo-[2,1-a]isoquinolines containing a pyridyl-, thienyl- or furyl ring in the 5-position, which compounds are useful as anorexics and anti-depressants. USP 4,101,553 discloses certain 5-hydroxy-2,3,5,6-tetrahydrofuran imidazo[2,1-a]isoquinolines containing an optionally substituted aryl group in the 5-position, said compounds useful as anorexics and anti-depressants.

The essence of the present invention is the discovery that certain 5-hetero- or aryl-substituted-imidazo-(2,1-a)-isoquinolines are useful as PAF receptor antagonists and in addition, that a select group thereof are useful as anti-tumor agents.

In one aspect therefore the invention concerns the use of compounds of formula I

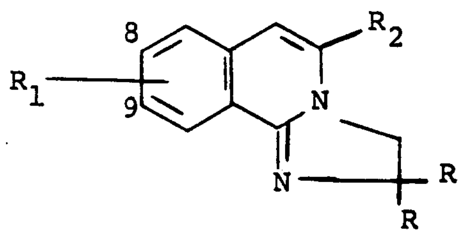

I

wherein each R independently is hydrogen or methyl,

$R_1$ is in 8- or 9-position and represents hydrogen, chloro, or $C_{1-3}$alkyl and

$R_2$ represents thienyl, furyl or a group

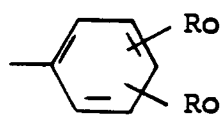

wherein each Ro represents independently hydrogen, fluoro, chloro, $C_{1-10}$alkyl or $C_{1-10}$alkoxy or one Ro is hydrogen and the other represents

a) tri-($C_{1-3}$alkyl)silyl, trifluoromethy or phenyl;

b) a group

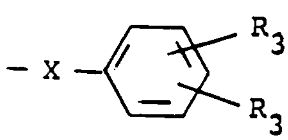

wherein each $R_3$ represents independently hydrogen, chloro, fluoro, $C_{1-4}$alkyl or $C_{1-5}$alkoxy and X represents

$-CH_2O-$, $-OCH_2$, $-CH_2-$, $-CH_2CH_2-$, or -O-;

c) a group

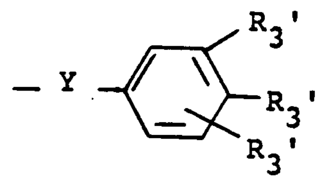

wherein each $R_3$' represents independently $C_{1-3}$alkoxy and
Y represents $-O(CH_2)_{1-5}-$, $-(CH_2)_{1-6}-$or $-CH_2OCH_2-$ ; or
d) a group

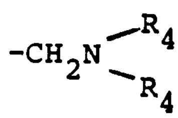

wherein each $R_4$ represents independently $C_{1-4}$alkyl, $C_{5-7}$cycloalkyl, allyl or benzyl; or the two $R_4$'s together with the nitrogen atom to which they are attached represent pyrrolidinyl, piperidyl, morpholinyl or thiomorpholinyl; or two Ro's on adjacent carbon atoms form methylenedioxy, with the proviso that when an Ro is other than chloro, fluoro, methyl or methoxy it may only be in a meta or para position and their pharmaceutically acceptable acid addition salts where such may exist, in the manufacture of a medicament for use in inhibiting PAF mediated bronchoconstriction and extravasation and PAF mediated endotoxin induced lung injury

Preferred compounds of formula I are those wherein each R is hydrogen, $R_1$ is as defined above and one Ro is hydrogen and the other is in meta or para position and is as defined under b) or c) above, and their pharmaceutically acceptable acid addition salts where such exist (compounds I').

Another aspect of the present invention concerns novel compounds of formula I i.e. compounds of formula Ia

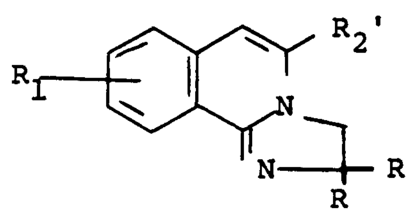

Ia

wherein R and $R_1$ are as defined above and
$R_2$' represents thienyl, furyl or a group

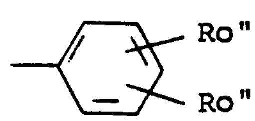

wherein one Ro" is hydrogen and the other represents tri-($C_{1-3}$alkyl)-silyl or phenyl, or is as defined under b), c) or d) above whereby Ro" substituents may only be in meta or para positions;
and their pharmaceutically acceptable acid addition salts where such may exist.

Preferred compounds of formula Ia are those wherein each R is hydrogen, $R_1$ is in 8-position and is otherwise as defined above and $R_2$ represents thienyl, furyl or a group

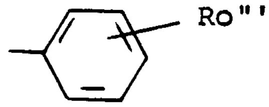

wherein Ro''' is in meta or para position and represents tri-($C_{1-3}$alkyl)silyl or is as defined under b), c) or d) above, and their pharmaceutically acceptable acid addition salts where such may exist (compound Ia').

A further aspect of the invention concerns the use of compounds of formula Ib

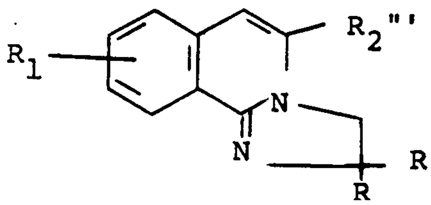

Ib

wherein R and $R_1$ are as defined above and
$R_2$''' represents thienyl, furyl or a group

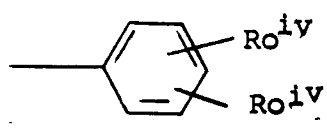

wherein each $Ro^{iv}$ represents independently, chloro, fluoro, or $C_{1-6}$alkyl or one $Ro^{iv}$ is hydrogen and the other is $C_2$-$C_6$alkyl tri-($C_{1-3}$alkyl) silyl, phenyl or is as defined under b) or c) above or is

d)' a group

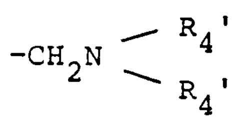

wherein each $R_4$' is allyl or the two $R_4$''s together with the nitrogen atom to which they are attached represent pyrrolidinyl, piperidyl, morpholinyl or thiomorpholinyl, with the proviso that when $Ro^{iv}$ is other than chloro, fluoro or methyl it may only be in meta- or para-position

and their pharmaceutically acceptable acid addition salts, where such may exist for use in the manufacture of a medicament for use in treating tumors.

Preferred compounds of formula Ib are those wherein R and $R_1$ are as defined above and $R_2$''' represents thienyl, furyl or a group

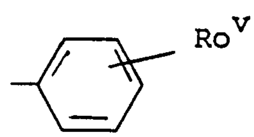

wherein $Ro^{v}$ is in meta or para position and represents $C_{2-6}$alkyl, tri($C_{1-3}$alkyl)silyl, phenyl, is as defined under b) or c) above or represents d)" a group

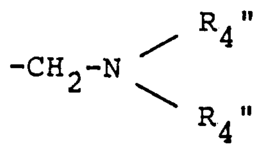

wherein each $R_4$" is allyl or the two $R_4$" 's together with the nitrogen atom to which they are attached represent pyrrolidinyl or piperidyl; and their pharmaceutically acceptable acid addition salts, where such may exist (compound Ib').

Alkyl moieties may be straight chained or branched.

A particular compound group covers those of formula I (and analogously for Ia and Ib) wherein

$R_1$ is as defined above and

$R_2$ is thienyl, furyl or a group

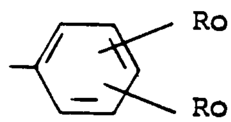

wherein each Ro represents independently hydrogen, fluoro, chloro, $C_{1-10}$alkyl, $C_{1-10}$alkoxy or $C_{1-10}$-alkylthio or one is hydrogen and the other is as defined under a) or d) above or

b)' a group

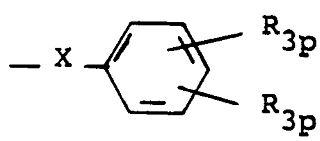

wherein each $R_{3p}$ represents independently hydrogen, chloro, fluoro or $C_{1-3}$alkoxy and X is as defined above or

c)' a group

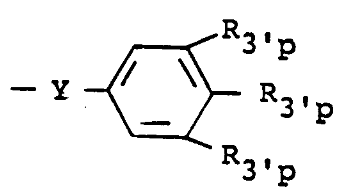

wherein each $R_{3'p}$ represents $C_{1-3}$alkoxy and Y represents $-OCH_2$, $-CH_2-$, $-CH_2CH_2-$ or $CH_2OCH_2$, and their pharmaceutically acceptable acid addition salts where such may exist.

In another compound group of formula I (and analogously for Ia and Ib)

$R_1$ is in 8- or 9-position and represents hydrogen, fluoro or $C_{1-3}$alkyl and

$R_2$ represents thienyl, furyl or a group

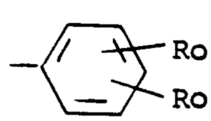

wherein each Ro represents independently hydrogen, fluoro, chloro, $C_{1-10}$alkyl or $C_{1-10}$alkoxy or one Ro is hydrogen and the other represents

a) tri-($C_{1-3}$alkyl)silyl, trifluoromethyl or phenyl

b) a group

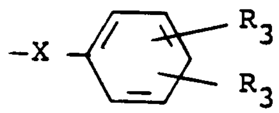

wherein each $R_3$ represents independently hydrogen, chloro, fluoro or $C_{1-5}$alkoxy and X represents-$OCH_2$-,-O-,-$CH_2O$- or -$CH_2CH_2$-or

c) a group

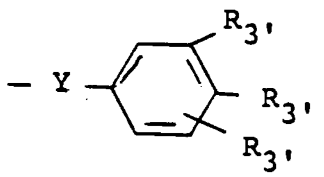

wherein each $R_3$, represents independently $C_{1-3}$alkoxy and Y represents-$OCH_2$-,-$CH_2OCH_2$-,-$CH_2$-,-$CH_2CH_2$- or -$CH_2CH_2CH_2$- or

d) a group

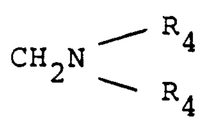

wherein each $R_4$ represents independently $C_{1-4}$alkyl, $C_{5-7}$cycloalkyl, allyl or benzyl or the two $R_4$'s together with the nitrogen atom to which they are attached represent pyrrolidinyl, piperidyl, morpholinyl or thiomorpholinyl or two Ro's on adjacent carbon atoms stand for methylenedioxy, with the proviso that when an Ro is other than chloro, fluoro, methyl or methoxy it may only be in a meta- or para-position.

The compounds of formula I may be prepared by dehydrating a compound of formula V

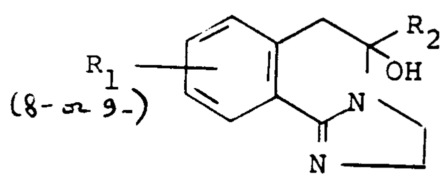

V

wherein $R_1$ and $R_2$ are as defined above and recovering the compound obtained in free form or in the form of a pharmaceutically acceptable acid addition salt where such may exist.

This reaction is carried out in an inert organic solvent and in the presence of an acid catalyst at temperatures e.g. of 35° to 200°C especially between 75° and 120°C. Examples of suitable solvents are aliphatic hydrocarbons e.g. hexane, heptane and the like, aromatic hydrocarbons e.g. benzene, toluene and the like, chlorinated hydrocarbons e.g. chloroform, methylenechloride and the like, aliphatic ethers e.g. diethyl ether, cyclic ethers e.g. tetrahydrofuran or an excess of acid catalyst e.g. acetic acid. Examples of suitable acid catalysts are mineral acids e.g. hydrochloric acid, sulphuric acid, phosphoric acid and the like or organic acids such as arylcarboxylic acids e.g. benzoic acid, alkylsulphonic acids e.g. methanesulphonic acid, aryl sulphonic acids e.g. p-toluenesulphonic acid or preferably alkylcarboxylic acids especially acetic acid. The invention also concerns a process for preparing compounds of formula Ia.

The compound of formula V may be prepared by reacting a compound of formula III

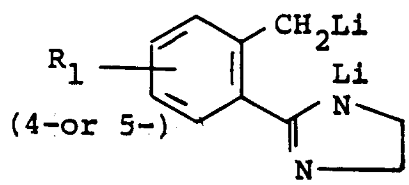

III

with a compound of formula IV

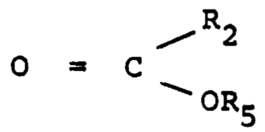

IV

wherein $R_1$ and $R_2$ are as defined above and $R_5$ represents $C_{1-4}$alkyl and hydrolysing the adduct thus formed and recovering the compound obtained in free form or in the form of a pharmaceutically acceptable acid addition salt.

The reaction is carried out under nitrogen atmosphere in an inert solvent such as an aliphatic hydrocarbon e.g. hexane, heptane and the like, an aliphatic ether e.g. diethyl ether or a cyclic ether e.g. tetrahydrofuran at temperatures of e.g. -30° to 50°C preferably -20° to 0°C. Hydrolysis is conducted in conventional manner e.g. employing water, dilute mineral acid, ammonium chloride solution or the like.

The compounds of formula III can be prepared by reacting a compound of formula II

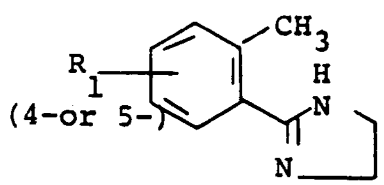

II

with $R_5Li$ wherein $R_1$ and $R_5$ are as defined above.

The reaction is carried out under a nitrogen atmosphere in an inert solvent such as described above for the reaction of III with IV at a temperature of e.g. 25° to 75°C preferably 30° to 40°C.

Certain compounds of formula V are new and also form part of the invention.

The invention thus also concerns compounds of formula Va

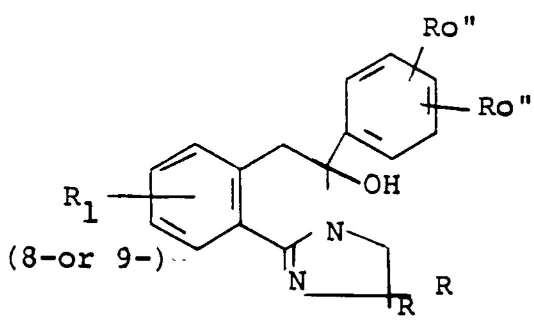

Va

wherein R, $R_1$ and each Ro" are as defined above and their pharmaceutically acceptable acid addition salts.

The starting materials of formula II and IV are either known and/or may be obtained analogously to known methods in conventional manner.

Final products and intermediates may be isolated and purified in conventional manner. Intermediates, where appropriate may be employed directly in the following step without purification.

As is evident to those skilled in the art, the compounds of formulae I and V may exist in racemic or enantiomeric form and the invention is intended to cover all forms.

Enantiomeric forms may be recovered in conventional manner e.g. by resolution of end or intermediate

products or by employing optically active starting materials.

Examples of pharmaceutically acceptable acid addition salts include those with mineral acids e.g. hydrochloric, hydrobromic, phosphonic and sulphuric and organic acids e.g. tartaric, acetic, citric, malic, maleic, methanesulphoric and gluconic which may be prepared in conventional manner.

The compounds of formula I are indicated for use as platelet activating factor receptor antagonists as indicated by their ability to inhibit specific binding of [$^3$H]-PAF to platelets according to the Human Platelet PAF Receptor Assay test (Test A) as follows:

Human blood is obtained by venipuncture of healthy, human donors into an anti-coagulant mixture containing 3.15% of trisodium citrate and 20 ug/ml of Prostaglandin $I_2$ ($PGI_2$) in a ratio of blood to anti-coagulant of 9:1. Platelet rich plasma (PRP) is prepared by centrifugation (250 x g) of the blood for 20 minutes at room temperature. The PRP is then centrifuged (900 x g) for 10 minutes at room temperature and the platelet pellet is washed two times with Tris-Tyrode's (TT) solution having a pH of 7.4 and containing 0.25% bovine serum albumin (BSA), and to which has been added $PGI_2$ at a final concentration of 0.3 $\mu$g/ml. The platelets are resuspended at 350,000 $\mu$l in TT/BSA containing 1.4 mM $CaCl_2.2H_2O$ and 0.7 mM $MgCl_2.6H_2O$. All of the tests are conducted in duplicate and each of the test compounds is evaluated at concentrations of 100, 50, 1 and 0.1 $\mu$M. For each determination, the following solutions are mixed:

500 $\mu$l of the above-described platelets;
10 $\mu$l of [$^3$H]-PAF (40,000 counts per minute (cpm) to a final concentration of 1.5 $\mu$M); and

either

10 $\mu$l of the test compound at 50x the desired final concentration,
10 $\mu$l of vehicle (total bound), or
10 $\mu$l of 1.85 x $10^{-5}$ M cold PAF (non-specifically bound).

Each mixture is allowed to incubate at room temperature for one hour, after which time the reaction is terminated by the addition of 500 $\mu$l of ice cold TT/BSA and centrifugation (900 x g) at 4°C for 10 minutes. The resultant supernatant is aspirated into scintillation vials and the pellet is washed with 250 ml of ice cold TT/BSA and centrifuged (900 x g) at 4°C for 10 minutes. The supernatants are then aspirated into the same scintillation vials as before and 10 ml of Scintiverse II (a liquid scintillation cocktail) is added to and mixed therewith. The pellets are resuspended in 500 $\mu$l of Scintiverse II and mixed well. An additional 2 ml of Scintiverse II is then added to the vials and, after mixing, the vials are counted for 1 minute in a liquid scintillation spectrometer. The amount of specific binding is calculated as the difference in cpm between the total bound [$^3$H]-PAF and non-specifically bound [$^3$H]-PAF. The percent inhibition of specific binding is determined by dividing the cpm specifically bound in the presence of the test compound by the cpm specifically bound in total, multiplying by 100 and then subtracting from 100. An $IC_{50}$ (50% inhibitory concentration) value is generated by evaluating the test compound over the full concentration range.

Furthermore, in view of their potential as PAF receptor antagonists, the compounds of formula I are indicated for use as inhibitors of PAF-mediated bronchoconstriction, which property was evaluated by the PAF-induced Pulmonary Inflation Pressure (PIP) Increase test (Test 8) as follows:

Male guinea pigs, weighing between 300 and 400 g, are anesthetized, after which time trachea tube, carotid and jugular catheters are inserted. The test animal is then force ventilated employing a small animal Harvard respirator and the resistance to lung inflation (PIP) is measured utilizing a pressure transducer and recorder. The test compound is administered either orally at 30 minutes up to 4 hours prior to or parenterally e.g. intravenously (jugular) etc. at 5 minutes up to 4 hours prior to the introduction of PAF. The PAF ($C_{18}$-Sandoz, Hanover) is dissolved in Tris-Tyrode's bovine serum albumin buffer and administered intravenously (jugular) at 100 ng/kg. Any blood pressure measurements taken are recorded from a transducer attached to the carotid catheter. Two responses are noted in the PIP recordings after the PAF is administered: 1) an immediate response which, in PAF-only treated test animals, averages out to between 70% and 80% more than the baseline PIP values. (This early response is also the greatest response and is, therefore, termed maximal PIP); and 2) the long term (at least 30 minutes) PIP response which slowly decreases to baseline. A reading at 15 minutes after the administration of PAF is termed the endpoint PIP. The effect of the test compound on the PIP response is determined by the difference between the percent increase in maximal PIP over baseline for the test animal to which has been administered PAF and the test compound compared to the test animal to which only PAF has been administered.

Still further, the compounds of formula I are indicated for use as inhibitors of PAF-mediated extravasation (the extrusion of plasma from the lumen of the blood vessels into the vessel wall and surrounding tissues) measured as a function of hemoconcentration according to the PAF-induced Extravasation test(Test C) as follows:

Male guinea pigs, weighing between 300 and 400 g, are anesthetized, after which time a femoral

catheter is inserted. The test compound is administered either orally or parenterally one hour up to 4 hours prior to the introduction of PAF. The PAF ($C_{18}$-Sandoz, Hanover) is dissolved in Tris-Tyrode's bovine serum albumin buffer and administered intravenously (jugular) at 100 ng/kg.

To determine the hematocrit value, which is employed to index hemoconcentration and is defined as the percent of packed red blood cells in a sample of blood which is centrifuged to separate plasma from the cellular components, blood samples are collected in 50 µl heparinized hematocrit tubes. These samples are taken just prior to the injection of PAF, one minute subsequent to the injection of PAF and every two minutes thereafter until 15 minutes has lapsed subsequent to the injection of PAF. The tubes are then centrifuged and the percent of packed red blood cells (hematocrit) is measured (PAF induces a maximal increase in hematocrit at 5 to 7 minutes subsequent to the injection of PAF). The percent increase in hematocrit over the value prior to the injection of PAF is calculated. The hemacrotit values obtained with the test compound are compared to the hemoconcentration values obtained with PAF alone and are expressed as percent inhibition of percent increase in hematocrit.

Yet still further, the compounds of formula I are indicated for use as inhibitors of PAF-mediated, endotoxin-induced lung injury and, analogously, endotoxin-induced-septic shock and adult respiratory distress syndrome. The term endotoxin induced lung injury is used generically for all three indications. The ability of the compounds of formula I to inhibit PAF-mediated, endotoxin-induced lung injury can be measured in accordance with the test presented by S. Chang at the 2nd International Conference on Platelet Activating Factor and Structurally Related Alkyl Ether Lipids in Gatlinburg, Tennessee on October 26-29, 1986.

Based on previous reports that lung tissue and blood PAF increased in endotoxin-treated rats, it was determined that the intraperitoneal administration of 2mg/kg of endotoxin (S. enteritidis) causes acute lung injury, as assessed by the extravascular accumulation of water $^{125}$I-albumin in perfused lungs isolated from rats ninety minutes following in vivo endotoxin treatment. Thus, the wet lung/body weight ratio (as an index of lung water content) inceases from 5.33 ± 0.48 to 8.26 ± 0.36 and the albumin leak index increases from 0.46 ± 0.09 to 1.01 ± 0.07. In order to measure the effectiveness of a compound as an inhibitor of endotoxin-induced lung injury, the test compound is administered intraperitoneally prior to the in vivo endotoxin treatment.

The ability of the compounds of formula I to inhibit PAF-mediated, endotoxin-induced septic shock can be measured in accordance with the test presented by C.N. Sessler, et al at the Annual Meeting of the American Federation for Clinical Research in New Orleans, Louisiana during January, 1987.

All sheep are prepared for testing employing the Chronic Sheep Lung Lymph preparation which is well documented in the literature, with the modifications that chronic tracheostomies are performed on the test animals and pleural pressure catheters inserted at the time of the initial surgery. All catheters are brought to the outside through stab wounds in the skin, the chest is closed and the test animals are allowed to recover for several days until they appear healthy and lung lymphs are free of blood before experiments are commenced.

In order to measure the effectiveness of a compound as an inhibitor of some of the important hemodynamic effects of endotoxin on septic shock, 1.3µg/kg of endotoxin or saline is administered to groups of test animals intravenously over a 30 minute period and 20mg/kg of the test compound or saline is administered intravenously over a five-hour period. The pulmonary arterial pressure (PAP), cardiac output (CO) and partial oxygen pressure ($PO_2$) are monitored continuously over the five-hour period.

The ability of the compounds of formula I to inhibit PAF-mediated, endotoxin-induced adult respiratory distress syndrome can be measured in accordance with the test presented by B.W. Christman, et al at the Annual Meeting of the American Thoracic Society and American Lung Association on May 10th -13th, 1987.

In order to measure the effectiveness of a compound as an inhibitor of some of the important hemodynamic effects of endotoxin on adult respiratory distress syndrome, 0.5µg/kg of E.coli endotoxin over a 20 minute period, 20mg/kg/hr of the test compound for 6 hours, or 0.5µg/kg of E.coli endotoxin 1 hour after commencing 20mg/kg/hr of the test compound for 6 hours, are administered to groups of test animals intravenously. The pulmonary arterial pressure (PAP), dynamic compliance (DC) of the lungs and lung lymph flow (LLF) are monitored continuously over a five-hour period.

The compounds are thus indicated for use in treating PAF mediated bronchoconstriction and extravasation and PAF mediated endotoxin induced lung injury and an indicated suitable daily dosage for this use is from about 10 to 2000 mg preferably 10 to 350 mg suitably administered in divided dosages of 0.25 to 500 mg (esp. 0.25 to 350 mg) up to four times daily or in controlled release form. A typical oral dosage is 50 or 100 mg two or three times a day. A typical oral unit dosage may contain 2.5 to 500 mg preferably 5 to 200 mg especially 10 to 100 mg of active substance.

The compounds of formula V are also indicated for use in the same indications and can be employed at

similar dosages.

The compounds of formula Ib are also indicated for use as anti-tumor agents as evidenced by their ability in inhibiting various lymphoma, sarcoma, myeloma and leukemia cell lines as indicated in the following tests.

Test D) Tumor cell cytotoxicity Test (TCCT).

In flat bottom microtiter plates (Nunc Roskieide, Denmark) were placed Abelson 8.1 tumor cells in DMEM + 10% fetal calf serum and the tumor cell-containing plates were incubated with 1,3, and 5µg of the test compound for a period of 6 to 72 hours. The number of viable tumor cells present was determined by measuring the alkaline phosphatase in the following manner. The tumor cell plates were centrifuged (500 x g.) for ten minutes and the supernatant flicked off. Without further washing, 100 µl of buffer containing 20 µl of diethanolamine, 2µM of $MgCl_2 \cdot 6H_2O$, 2.5 µM of p-nitrophenylphosphate and 10 mg Triton X-100 were added. The samples were incubated for 60 minutes at room temperature and the enzymatic reaction was terminated by the addition of 100 µl of 0.5N NaOH. The absorbance was then measured at 405 nM using a Titertek Multiskan apparatus.

Test E) Influence on Cytotoxicity of ET-18-$OCH_3$ test (IC-ET test).

Bone marrow cell macrophages ($10^5$/well)obtained from [BALB/CX57/$BL_6$]Fl mice were incubated with 10µg of 1-octadecyl-2-methoxy-3-phosphoryl choline (ET-18-$OCH_3$) for 24 hours in flat bottom microtiter plates (Nunc Roskieide, Denmark), after which time they are centrifuged and washed once. Abelson 8.1 tumor cells in DMEM + 10% fetal calf serum and 1,3 and 5µg of the test compound were then added to the plates. With the cytotoxicity of ET-18-$OCH_3$ (10µg) alone set at 100%, the inhibition or enhancement of the cytotoxic effect, as measured by an alkaline phosphatase assay, was determined and values recorded after 72 hours for 1,3 and 5µg of the test substance.

Test F) Meth A fibrosarcoma cells were induced in BALB/C mice by administering methylcholanthrene according to the procedure of Old, et al. (L.J. Old, E.A. Boyse, D.A. Clarke, and E. Carswell, Ann. N.Y. Acad. Sci., 101, 80 (1962). These tumor cells were harvested from the peritoneal cavity 10 to 12 days after administration of methylcholanthrene. Ten $CBF_1$ mice of 10-12 week age were each implanted with 7.3 x $10^6$ Meth A sarcoma cells to serve as control. A second group of ten $CBF_1$ mice were each implanted with 7.3 x $10^6$ Meth A sarcoma cells and on day one after implant each mouse was treated p.o. with 5-50µg of the test compound per day for a total of twenty or twenty-seven days. Tumor growth and survivors were assayed on days 7, 14, 21 and 28 after tumor implantation. Under these conditions, none of the control group animals survived, whereas all of the animals which were administered 50µg per day of the compound of Example 3N) for 28 days not only survived but showed no evidence of the presence of any tumors as well.

The compounds are thus further indicated for use in inhibiting tumors and an indicated suitable dosage for this use is from about 500-2000 mg preferably 1000 - 1500 mg administered in divided dosages of e.g. 125 to 750 mg up to 4 times daily or in controlled release form. A typical oral dosage is 400 mg two or three times a day or 20 mg/kg body weight intravenously over a 24 hours period. A typical oral unit dosage may contain 300 to 600 mg preferably 300 to 500 mg especially 350 to 450 mg of active substances.

A suitable daily dosage will depend on a number of factors such as relative potency of activity of the compound. The preferred compound according to the invention 5-[4'-(3,4,5-trimethoxyphenylethyl)phenyl]-2,3-dihydroimidazo[2,1-a]-isoquinoline hydrochloride achieved e.g. the following results in tests A to C, Test A -$IC_{50}$ 0,06 µM, Test B $ED_{50}$ 5.0 mg/kg p.o., Test C -$ED_{50}$ 4.2 mg/kg p.o. The compound 5-(4'-diallylaminomethylphenyl)-2,3-dihydroimidazo[2,1-a]isoquinoline hydrochloride achieved e.g. the following results in tests D and E : Test D - 99% inh.at 5µg, Test E - 97% inh. at 5µg.

The invention therefore also concerns a the use of a compound of formula I or a pharmaceutically acceptable salt thereof in the manufacture of medicaments for treating PAF mediated bronchoconstriction and extravasation and PAF-mediated endotoxin induced lung injury.

The invention further concerns the use of a compound of formula Ib or a pharmaceutically acceptable salt thereof in the manufacture of medicaments for use as anti-tumor agents.

The compounds may be employed in free form or in the form of pharmaceutically acceptable acid addition salts where such exist.

The compounds of the invention, in free form or in pharmaceutically acceptable acid addition salt form may be combined with one or more pharmaceutically acceptable carriers and, optionally, one or more other conventional pharmaceutical adjuvants and administered orally in the form of tablets, dispersible powders,

granules, capsules, elixirs, suspensions and the like or parenterally in the form of sterile injectable solutions or suspensions. The compositions may be prepared by conventional means.

Examples of compositions are as follows:

| Solid unit dosage forms (weight (mg) ) | | | | |
|---|---|---|---|---|
| | tablet | | capsule | |
| Compound of formula I (e.g. of Ex. 7E) | 50 | - | 50 | - |
| Compound of formula Ib (e.g. of Ex 3N) | - | 400 | - | 400 |
| tragacanth | 10 | 10 | - | - |
| lactose (spray-dried) | 212.5 | 197.5 | 100 | 250 |
| corn starch | 15 | 25 | - | - |
| talcum | 10 | 15 | - | - |
| magnesium stearate | 2.5 | 2.5 | - | - |
| Total | 300.0 | 650.0 | 150.0 | 650.0 |

| Liquid forms (weight (mg) ) | | |
|---|---|---|
| Ingredients | sterile injectable suspension | oral liquid suspension |
| compound of formula I, e.g., the compound of Example 7E ) | 5 | 3 |
| sodium carboxymethylcellulose | U.S.P 1 | 8 |
| methyl cellulose | 0.3 | - |
| polyvinylpyrrolidone | 2.7 | - |
| lecithin | 1.5 | - |
| benzyl alcohol | 0.01 | - |
| magnesium aluminum silicate | - | 25 |
| flavor | - | q.s. |
| color | - | q.s. |
| methyl paraben, U.S.P | - | 3 |
| propyl paraben, U.S.P. | - | 0.7 |
| polysorbate 80 (e.g., Tween 80), | U.S.P. - | 5 |
| sorbitol solution, 70%, U.S.P. | - | 1450 |
| buffer agent to adjust pH for desired stability | q.s. for injection | q.s. |
| water | q.s. to 1 ml | q.s. to 5 ml |

The preferred pharmaceutical compositions from the standpoint of preparation and ease of administration are solid compositions, particularly liquid or hard-filled capsules and tablets containing from about 10 to 100 mg of the active ingredient concerning the PAF inhibition use and from about 350 to 450 mg of the active ingredient with respect to tumor inhibition.

Such compositions also form part of the invention.

The following examples, in which all temperatures are in ° C illustrate the invention.

Example 1

5-(p-fluorophenyl)-2,3-dihydroimidazo-[2,1-a]isoquinoline($R_1$ = H, $R_2$ = p-fluorophenyl).

a) 5-(p-fluorophenyl)-2,3,5,6-tetrahydroimidazo[2,1-a] isoquinoline-5-ol (Compound of formula V in wherein $R_1$ = H, $R_2$ = p-fluorophenyl).

Into a flask, maintained under nitrogen and equipped with a stirrer, condenser, and dropping funnel, are charged 6.26 g (0.04 mol) off 2-(o-tolyl)-2(1H)-imidazoline and 100 ml of dry tetrahydrofuran. The solution is stirred, treated with 84 ml of 1.6M n-butyl lithium (0.13 mol) in hexane and maintained at 35° C for ca. 5 hours. The mixture is treated with 20.7 g (0.16 mol) of ethyl-p-fluorobenzoate in 50 ml of dry tetrahydrofuran and then maintained at 50° C for ca. 5 hours. The reaction is, then cooled in an icebath and treated with

22.8 ml of saturated ammonium chloride. The tetrahydrofuran layer is separated in a separatory funnel, dried with magnesium sulfate, filtered and then concentrated in vacuo. The residue is dissolved in a hot methylene chloride-methanol (1:1) mixture which, upon cooling, yields the desired compound;

b) 5-(p-fluorophenyl)-2,3-dihydroimidazo-[2,1-a]isoquinoline

A solution containing 2.8 g (0.01 mol) of the compound prepared in a) above and 40 ml of glacial acetic acid is refluxed, with stirring, under a nitrogen atmosphere for ca. 5 hours. The acetic acid is then removed by evaporation in vacuo and the residue treated with 50 ml of water and then made alkaline with 35 ml of 2N sodium carbonate. The mixture is then extracted with two 100 ml portions of methylene chloride, after which time the organic layer is separated, dried over anhydrous magnesium sulfate, filtered and the solvent removed in vacuo. The crude product obtained is recrystallized employing a mixture of methanol and water to yield the title compound, m.p. 150°-151°C.

Example 2

Following essentially the procedure of Example 1a) above, and using in place of the ethyl-p-fluorobenzoate, the appropriate compound of formula IV, the following compounds of formula V are obtained:

A) 5-[4-diethylamino)methylphenyl]-2,3,5,6-tetrahydroimidazol[2,1-a]isoquinolin-5-ol;
B) 5-(p-chlorophenyl)-2,3,5,6,tetrahydroimidazo [2,1-a]isoquinolin-5-ol;
C) 5-phenyl-2,3,5,6-tetrahydroimidazo[2,1-a]isoquinolin-5-ol;
D) 5-(3,4-dimethoxyphenyl)-2,3,5,6-tetrahydroimidazo[2,1-a]isoquinolin-5-ol;
E) 5-(4-methoxyphenyl)-2,3,5,6-tetrahydroimidazo[2,1-a]isoquinolin-5-ol;
F) 5-(3,4-methylenedioxyphenyl)-2,3,5,6-tetrahydroimidazo[2,1-a]isoquinolin-5-ol;
G) 5-(3,4-dichlorophenyl)-2,3,5,6-tetrahydroimidazo[2,1-a]isoquinolin-5-ol;
H) 5-(2,4-dichlorophenyl)-2,3,5,6-tetrahydroimidazo[2,1-a]isoquinolin-5-ol;
I) 5-(3-tritluoromethylphenyl)-2,3,5,6-tetrahydroimidazo[2,1-a]isoquinolin-5-ol;
J) 5-(2-chlorophenyl)-2,3,5,6-tetrahydroimidazo[2,1-a]isoquinolin-5-ol;
K) 5-(4-methylphenyl)-2,3,5,6-tetrahydroimidazo(2,1-a]isoquinolin-5-ol;
L) 5-(3-chlorophenyl-2,3,5,6-tetrahydroimidazo[2,1-a]isoquinolin-5-ol;
M) 5-(2-fluorophenyl)-2,3,5,6-tetrahydroimidazo[2,1-a]isoquinolin-5-ol;
N) 5-(4-piperidinomethyiphenyl)-2,3,5,6-tetrahydroimidazo[2,1-a]isoquinolin-5-ol;
O) 5-(4-pyrrolidinomethylphenyl)-2,3,5,6-tetrahydroimidazo[2,1-a]isoquinolin-5-ol;
P) 5-(4-morpholinomethylphenyl)-2,3,5,6-tetrahydroimidazo[2,1-a]isoquinolin-5-ol;
Q) 5-(2-thienyl)-2,3,5,6-tetrahydroimidazo[2,1-a] isoquinolin-5-ol;
R) 5-[4'-(2-chloro-4-fluorobenzyloxy)phenyl]-2,3,5, 6-tetrahydroimidazo[2,1-a]isoquinolin-5-ol;
S) 5-(4'-(2,6-dichlorobenzyloxy)phenyl)-2,3,5,6-tetrahydroimidazo[2,1-a]isoquinolin-5-ol;
T) 5-(4-t-butylphenyl)-2,3,5,6-tetrahydroimidazo [2,1-a]isoquinolin-5-ol;
U) 5-(4-phenylphenyl)-2,3,5,6-tetrahydroimidazo [2,1-a]isoquinolin-5-ol;
V) 5-(4-dibenzylaminomethylphenyl)-2,3,5,6-tetrahydroimidazo[2,1-a]isoquinolin-5-ol;
W) 5-(4'-dicyclohexylaminomethylphenyl)-2,3,5,6-tetrahydroimidazo[2,1-a]isoquinolin-5-ol;
X) 5-(4'-diisopropylaminomethylphenyl)-2,3,5,6-tetrahydroimidazo[2,1a]isoquinolin-5-ol;
Y) 5-(4'-ethylphenyl)-2,3,5,6-tetrahydroimidazo [2,1-a]isoquinolin-5-ol;
Z) 5-(4'-trimethylsilylphenyl)-2,3,5,6,-tetrahydroimidazo[2,1-a]isoquinolin-5-ol;
AA) 5-(4'-phenoxyphenyl)-2,3,5,6-tetrahydroimidazo [2,1-a]isoquinolin-5-ol;
BB) 5-(4'-diallylaminomethylphenyl)-2,3,5,6-tetrahydroimidazo[2,1-a]isoquinolin-5-ol;
CC) 5-(4'-benzyloxyphenyl)-2,3,5,6-tetraffydroimidazo [2,1-a]isoquinolin-5-ol;
DD) 5-(2'-furyl)-2,3,5,6-tetrahydroimidazo[2,1-a] isoquinolin-5-ol;
EE) 5-(4'-thiomorpholinomethylphenyl)-2,3,5,6-tetrahydroimidazo[2,1-a]isoquinolin-5-ol;
FF) 5-(3'-t-butylphenyl)-2,3,5,6-tetrahydroimidazo [2,1-a]isoquinolin-5-ol;
GG) 5-[4'-(2,6-dichlorophenyloxymethyl)phenyl]-2,3,5,6-tetrahydroimidazo[2,1-a]isoquinolin-5-ol;
HH) 5-[4'-(3,4,5-trimethoxybenzyloxy)phenyl]-2,3,5,6-tetrahydroimidazo[2,1-a]isoquinolin-5-ol;
II) 5-[4'(2-fluorobenzyloxy)phenyl]-2,3,5,6-tetrahydroimidazo[2,1-a]isoquinolin-5-ol;
JJ) 5-[4'-(4-fluorobenzyloxy)phenyl]-2,3,5,6-tetrahydroimidazo[2,1-a]isoquinolin-5-ol;
KK) 5-[4'-(2-chlorobenzyloxy)phenyl]-2,3,4,5,6-tetrahydroimidazo[2,1-a]isoquinolin-5-ol;
LL) 5-[4'-(2-chloro-6-flurobenzyloxy)phenyl]-2,3,5,6-tetrahydroimidazo[2,1-a]isoquinolin-5-ol;
MM) 5-[4'-(2,4-dichlorobenzyloxy)phenyl]-2,3,5,6-tetrahydroimidazo[2,1-a]isoquinolin-5-ol;
NN) 5-[4'-(4-t-butylbenzyloxy)phenyl]-2,3,5,6-tetrahydroimidazo[2,1-a]isoquinolin-5-ol;

OO) 5-[4'-(3,4,5-trimethoxyphenylproppyloxy)phenyl]-2,3,5,6-tetrahydroimidazo[2,1-a]isoquinolin-5-ol;
PP) 5-[4'-(3,4,5-trimethoxyphenylhexyl)phenyl]-2,3,5,6-tetrahydroimidazo[2,1-a]isoquinolin-5-ol; and
QQ) 5-[4'-(3,4,5-trimethoxyphenylethyloxy)phenyl]-2,3,5,6-tetrahydroimidazo[2,1-a]isoquinolin-5-ol, respectively.

Example 3

Following essentially the procedure of Example Ib, and using in place of the compound of Example 1a), a suitable amount of a compound of Examples 2A) through 2NN), respectively, there are obtained:

A) 5-[4'-(diethylamino)methylphenyl)-2,3,-dihydroimidazo[2,1-a]isoquinoline dihydrochloride, m.p. 292°-294°C;
B) 5-(p-chlorophenyl)-2,3-dihydroimidazo[2,1-a] isoquinoline, m.p. >250°C;
C) 5-phenyl-2,3-dihydroimidazo[2,1-a]isoquinoline, m.p. 143°-145°C;
D) 5-(3',4'-dimethoxyphenyl)-2,3-dihydroimidazo[2,1-a]isoquinoline, m.p., 158°-160°C;
E) 5-(4'-methoxyphenyl)-2,3-dihydroimidazo [2,1-a]isoquinoline, m.p. 126°-128°C;
F) 5-(3',4'-methylenedioxyphenyl)-2,3-dihydroimidazo[2,1-a]isoquinoline, m.p. 128°-130°C;
G) 5-(3',4'-dichlorophenyl)-2,3-dihydroimidazo [2,1-a]isoquinoline, m.p. 158°-160°C;
H) 5-(2',4'-dichlorophenyl)-2,3-dihydroimidazo [2,1-a]isoquinoline, m.p. 150°-152°C;
I) 5-(3'-trifluoromethylphenyl)-2,3-dihydroimidazo[2,1-a]isoquinoline, m.p. >250°C;
J) 5-(2'-chlorophenyl)-2,3-dihydroimidazo [2,1-a]isoquinoline, m.p. 134°-136°C;
K) 5-(p-methylphenyl)-2,3-dihydroimidazo [2,1-a]isoquinoline, m.p. >250°C;
L) 5-(3'-chlorophenyl)-2,3-dihydroimidazo [2,1-a]isoquinoline, m.p. >250°C;
M) 5-(2'-fluorophenyl)-2,3-dihydroimidazo[2,1-a] isoquinoline, m.p. >250°C;
N) 5-(4'-piperidinomethylphenyl)-2,3-dihydroimidazo[2,1-a]isoquinoline dihydrochloride, m.p. 317°-320°C;
O) 5-[4'-pyrrolidinomethylphenyl)-2,3-dihydroimidazo[2,1-a]isoquinoline, m.p. 106°-108°C;
P) 5-(4'-morpholinomethylphenyl)-2,3-dihydroimidazo[2,1-a]isoquinoline,m.p. 160°-162°C;
Q) 5-[2-thienyl)-2,3-dihydroimidazo[2,1-a] isoquinoline, m.p. 98°-100°C;
R) 5-[4'-(2-chloro-4-fluorobenzyloxy)phenyl]-2,3-dihydroimidazo[2,1-a]isoquinoline hydrochloride, m.p. 252°-254°C. (dec.);
S) 5-[4'-(2,6-dichlorobenzyloxy)phenyl]-2,3-dihydroimidazo[2,1-a]isoquinoline hydrochloride, m.p. 277°-280°C;
T) 5-(4'-t-butylphenyl)-2,3-dihydroimidazo[2,1-a] isoquinoline, m.p. >200°C;
U) 5-(4'-phenylphenyl)-2,3-dihydroimidazo[2,1-a] isoquinoline, m.p. >200°C;
V) 5-(4'-dibenzylaminomethylphenyl)-2,3-dihydroimidazo[2,1-a]isoquinoline dihydrochloride, m.p. 225°C;
W) 5-(4'-dicyclohexylaminomethylphenyl)-2,3-dihydroimidazo[2,1-a]isoquinoline dihydrochloride, m.p. 273°C (dec.);
X) 5-(4 -diisopropylaminomethylphenyl)-2,3-dihydroimidazo[2,1-a]isoquinoline dihydrochloride, monohydrate, m.p. 264°C (dec.) ;
Y) 5-(4'-ethylphenyl]-2,3-dihydroimidazo[2,1-a]isoquinoline hydrochloride, m.p. 298°-300°C;
Z) 5-(4'-trimethylsilylphenyl)-2,3-dihydroimidazo [2,1-a]isoquinoline hydrochloride, m.p.>270°C;
AA) 5-(4'-phenoxyphenyl)-2,3-dihydroimidazo[2,1-a] isoquinoline hydrochloride, m.p.>270°C;
BB) 5-(4'-diallylaminomethylphenyl)-2,3-dihydroimidazo[2,1-a)isoquinoline dihydrochloride, m.p. 228°C;
CC) 5-(4'-benzyloxyphenyl)-2,3-dihydroimidazo[2,1-a] isoquinoline hydrochloride, m.p. 213°-215° C;
DD) 5-(2'-furyl)-2,3-dihydroimidazo[2,1-a]isoquinoline dihydrochloride, m.p.>315°C;
EE) 5-(4'-thiomorpholinomethylphenyl)-2,3-dihydroimidazo[2,1-a]isoquinoline dihydrochloride, m.p. 275°-278°C;
FF) 5-(3'-t-butylphenyl)-2,3-dihydroimidazo[2,1-a] isoquinoline hydrochloride, m.p. 290°C;
GG) 5-[4'-(2,6-dichlorophenyloxymethyl)phenyl)-2,3-dihydroimidazo[2,1-a]isoquinoline hydrochloride, m.p. 242°C;
HH) 5-[4'-(3,4,5-trimethoxybenzyloxy)phenyl]-2,3-dihydroimidazo[2,1-a]isoquinoline hydrochloride, m.p. 205°-207°C;
II) 5-[4'-(2-fluorobenzyloxy)phenyl]-2,3-dihydroimidazo[2,1-a]isoquinoline hydrochloride, m.p. 253°-254°C. (dec.);
JJ) 5-[4'-(4-fluorobenzyloxy)phenyl]-2,3-dihydroimidazo[2,1-a]isoquinoline hydrochloride, m.p. 257°-258°C (dec.);
KK) 5-]4'-(2-chlorobenzyloxy)phenyl]-2,3-dihydroimidazo[2,1-a[isoquinoline hydrochloride, m.p. 272°C. (dec.);

LL) 5-[4'-(2-chloro-6-fluorobenzyloxy)phenyl]-2,3-dihydroimidazo[2,1-a]isoquinoline hydrochloride, m.P.>250 ° C;

MM) 5-[4'(2,4-dichlorobenzyloxy)phenyl]-2,3-dihydroimidazo[2,1-a]isoquinoline hydrochloride, m.p. 254 ° C. (dec.);

NN) 5-[4'-(4-t-butylbenzyloxy)phenyl]-2,3-dihydroimidazo[2,1-a]isoquinoline hydrochloride, m.p. 271 ° c. (dec.);

OO) 5-[4'-(3,4,5-trimethoxyphenylpropyloxy)phenyl]-2,3-di hydroimidazo[2,1-a]isoquinoline hydrochloride, m.p. 215 ° -217 ° C. (dec);

PP) 5-[4'-(3,4,5-trimethoxyphenylhexyl)phenyl]-2,3-dihydroimidazo[2,1-a]isoquinoline hydrochloride, m.p. 213 ° -215 ° C;and

QQ) 5-[4'-(3,4,5-trimethoxyphenylethyloxy)phenyl]-2,3-dihydroimidazo[2,1-a]isoquinoline hydrochloride, m.p. 242 ° -243 ° C, respectively.

Example 4

Following essentially the procedure of Example 1a) above, and using in place of 2-(o-tolyl)-2(1H)-imidazoline and ethyl-p-fluorobenzoate, appropriate compounds of formulae III and IV, there are obtained:

A) 5-(4'-t-butylphenyl)-8-chloro-2,3,5,6-tetrahydroimidazo[2,1-a]isoquinolin-5-ol;

B) 5-(4'-t-butylphenyl)-9-chloro-2,3,5,6-tetrahydroimidazo[2,1-a]isoquinolin-5-ol;

C) 5-(4'-t-butylphenyl)-9-methyl-2,3,5,6-tetranydroimidazo[2,1-a]isoquinolin-5-ol;

D) 8-chloro-5-[4'-(3,4,5-trimethoxyphenylethyl)phenyl]-2,3,5,6-tetrahydroimidazo[2,1-a)isoquinolin-5-ol; and

E) 2,2-dimethyl-5-[4'-(3,4,5-trimethoxyphenylethyl)phenyl]-2,3,5,6-tetrahydroimidazo[2,1-a]isoquinolin-5-ol, respectively.

Example 5

Following essentially the procedure of Example 1b and using in place of the compound of Example 1a), a suitable amount of the compounds of Examples 4A) through 4C), respectively, there are obtained:

A) 5-(4'-t-butylphenyl)-8-chloro-2,3-dihydroimidazo [2,1-a]isoquinoline hydrochloride, m.p.>250 ° C;

B) 5-(4'-t-butylphenyl)-9-chloro-2,3-dihydroimidazo [2,1-a]isoquinoline hydrochloride, m.p.>298 ° C;

C) 5-(4'-t-butylphenyl)-9-methyl-2,3-dihydroimidazo [2,1-a]isoquinoline hydrochloride, m.p.>275 ° C;

D) 8-chloro-5[4'-(3,4,5-trimethoxyphenylethyl)phenyl]-2,3-dihydroimidazo[2,1-a]isoquinoline hydrochloride m.p.268 ° (dec); and

E) 2,2-dimethyl-5-[4'-(3,4,5-trimethoxyphenylethyl)phenyl]-2,3-dihydroimidazo[2,1-a]isoquinoline hydrochloride,

respectively.

Example 6

Following essentially the procedure of Example 1a) above, and using in place of the ethyl-p-fluorobenzoate,the appropriate compound of formula IV, there are obtained:

A) 5-[4'-(3,4,5-trimethoxybenzyloxymethyl)phenyl] - 2,3,5,6-tetrahydroimidazo[2,1-a]isoquinolin-5-ol;

B) 5-[4'-(3,4-dimethoxybenzyloxy)phenyl]-2,3,5, 6-tetrahydroimidazo[2,1-a]isoquinolin-5-ol;

C) 5-[3'-(3,4,5-trimethoxybenzyloxy)phenyl]-2,3,5, 6-tetrahydroimidazo [2,1-a]isoquinolin-5-ol;

D) 5-[2'-(3,4,5-trimethoxybenzyloxy)phenyl] -2,3,5,6-tetrahydroimidazo [2,1-a]isoquinolin-5-ol;

E) 5-[4'-(3,4,5-trimethoxyphenylethyl)phenyl]2,3,5, 6-tetrahydroimidazo [2,1-a]isoquinolin-5-ol;

F) 5-[3'-(3,4,5-trimethoxyphenylethyl)phenyl]-2,3,5, 6-tetrahydroimidazo [2,1-a]isoquinolin-5-ol;

G) 5-[4'-(3,4-dimethoxyphenylethyl)phenyl]2,3,5, 6-tetrahydroimidazo[2,1-a]isoquinolin-5-ol;

H) 5-[4'-(2-methoxyphenylethyl)phenyl]-2,3,5, 6-tetrahydroimidazo[2,1-a]isoquinolin-5-ol;

I) 5-[4'-(4-methoxybenzyloxy)phenyl]-2,3,5, 6-tetrahydroimidazo[2,1-a]isouqinolin-5-ol;

J) 5-[4'-(3,4-dimethylbenzyloxy)phenyl]-2,3,5, 6-tetrahydroimidazo [2,1-a]isoquinolin-5-ol;

K) 5-[4'-(3,4,5-trimethoxyphenylpropyl)phenyl]-2,3,5, 6-tetrahydroimidazo[2,1-a]isoquinolin-5-ol;

L) 5-[4'-(2,3,4-trimethoxybenzyloxy)phenyl]-2,3,5, 6-tetrahydroimidazo[2,1-a]isoquinolin-5-ol;

M) 5-[4'-(3-methoxy-4-pentoxyphenylethyl)phenyl]-2,3,5,6-tetrahydroimidazo[2,1-a]isoquinolin-5-ol; and

N) 5-[4'-(3,4,5-trimethoxybenzyl)phenyl]-2,3,5, 6-tetrahydroimidazo[2,1-a]isoquinolin-5-ol; respectively.

Example 7

Following essentially the procedure of Example 1b and using in place of the compound of Example 1a), a suitable amount of the compounds of Examples 6A) through 6N), respectively, there are obtained:

A) 5-[4'-(3,4,5-trimethoxybenzyloxymethyl)phenyl]-2,3-dihydroimidazo[2,1-a]isoquinoline hydrochloride, m.p. >240 ° C(dec.);

B) 5-[4'-(3,4-dimethoxybenzyloxy)phenyl]-2,3-dihydroimidazo[2,1-a]isoquinoline hydrochloride, monohydrate, m.p.>239 ° C(dec.);

C) 5-(3'-(3,4,5-trimethoxybenzyloxy)phenyl]-2, 3-dihydroimidazo[2,1-a]isoquioline hydrochloride, m.p. 230 ° C;

D) 5-[2'-(3,4,5-trimethoxybenzyloxy)phenyl]-2, 3-dihydroimidazo[2,1-a]isoquinoline hydrochloride, m.p. 252 ° C ;

E) 5-[4'-(3,4,5-trimethoxyphenylethyl)phenyl]-2, 3-dihydroimidazo [2,1-a]isoquinoline hydrochloride, m.p. 243 ° - 245 ° C ;

F) 5-[3'-(3,4,5-trimethoxyphenylethyl)phenyl]-2, 3-dihydroimidazo[2,1-a]isoquinoline hydrochloride, m.p. 260 ° - 261 ° C ;

G) 5-[4'-(3,4-dimethoxyphenylethyl)phenyl]-2, 3-dihydroimidazo[2,1-a]isoquinoline hydrochloride, m.p. 263 ° - 265 ° C;

H) 5-[4'-(2-methoxyphenylethyl)phenyl]2,3-dihydroimidazo[2,1-a]isoquinoline hydrochloride, m.p.>270 ° C ;

I) 5-[4'-(4-methoxybenzyloxy)phenyl]-2,3-dihydroimidazo[2,1-a]isoquinoline hydrochloride, m.p.>250 ° C ;

J) 5-[4'-(3,4-dimethylbenzyloxy)phenyl]-2,3-dihydroimidazo[2,1-a]isoquinoline hydrochloride, m.p.>270 ° C ;

K) 5-[4'-(3,4,5-trimethoxyphenylpropyl)phenyl]-2, 3-dihydroimidazo[2,1-a]isoquinoline hydrochloride, m.p. 242 ° - 244 ° C ;

L) 5-[4'-(2,3,4-trimethoxybenzyloxy)phenyl]-2, 3-dihydroimidazo[2,1-a]isoquinoline hydrochloride, m.p. >240 ° C ;

M) 5-(4'-(3-methoxy-4-pentoxyphenylethyl)phenyl]-2, 3-dihydroimidazo[2,1-a]isoquinoline hydrochloride, m.p. 260 ° C(dec.); and

N) 5-[4'-(3,4,5-trimethoxybenzyl)phenyl]-2, 3-dihydroimidazo[2,1-a]isoquinoline hydrochloride, m.p. 238 ° - 240 ° C,

respectively.

**Claims**

**Claims for the following Contracting States : AT, BE, CH, DE, FR, GB, IT, LI, LU, NL, SE**

**1.** Use of a compound of formula I

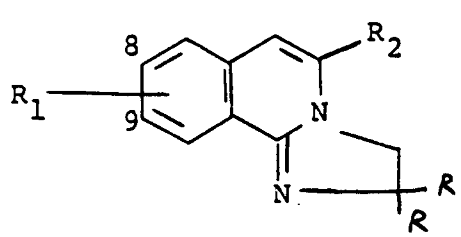

I

wherein each R independently is hydrogen or methyl $R_1$ is in 8- or 9-position and represents hydrogen, chloro, or $C_{1-3}$alkyl and
$R_2$ represents thienyl, furyl or a group

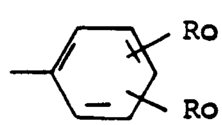

wherein each Ro represents independently hydrogen, fluoro, chloro, $C_{1-10}$alkyl or $C_{1-10}$alkoxy or one Ro is hydrogen and the other represents

a) tri-($C_{1-3}$alkyl)silyl, trifluoromethy or phenyl;

b) a group

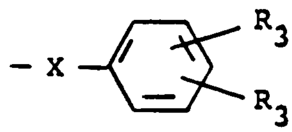

wherein each $R_3$ represents independently hydrogen, chloro, fluoro $C_{1-4}$alkyl or $C_{1-5}$alkoxy and X represents
$-CH_2O-$, $-OCH_2-$, $-CH_2-$, $-CH_2CH_2-$, or -O-;
c) a group

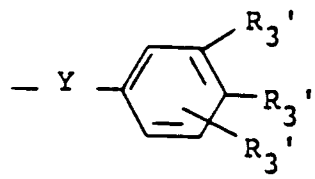

wherein each $R_3'$ represents independently $C_{1-3}$alkoxy and
Y represents $-(OCH_2)_{1-5}-$, $-(CH_2)_{1-6}-$ or $-CH_2OCH_2-$ ; or
d) a group

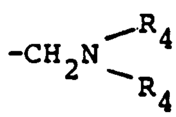

wherein each $R_4$ represents independently $C_{1-4}$alkyl, $C_{5-7}$cycloalkyl, allyl or benzyl; or the two $R_4$'s together with the nitrogen atom to which they are attached represent pyrrolidinyl, piperidyl, morpholinyl or thiomorpholinyl;
or two Ro's on adjacent carbon atoms form methylenedioxy, with the proviso that when an Ro is other than chloro, fluoro, methyl or methoxy it may only be in a meta or para position or a pharmaceutically acceptable acid addition salt thereof in the manufacture of a medicament for use in inhibiting PAF mediated bronchoconstriction and extravasation and PAF mediated endotoxin induced lung injury

**2.** The use according to claim 1 whereby in formula I each R is hydrogen, $R_1$ is as defined in claim 1 and one Ro is hydrogen and the other is in meta- or para-position and is as defined under b) or c) in claim 1.

**3.** Use of a compound of formula Ib

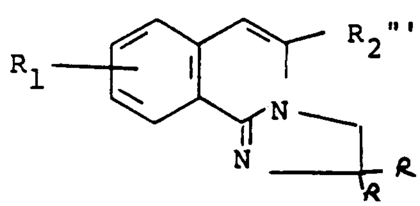

Ib

wherein R and $R_1$ are as defined in claim 1 and
$R_2'''$ represents thienyl, furyl or a group

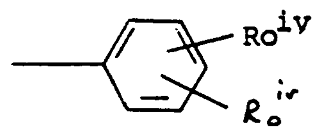

wherein each $Ro^{iv}$ represents independently chloro, fluoro or $C_{1-6}$alkyl or one $Ro^{iv}$ is hydrogen and the other is is in meta or para position and represents $C_{2-6}$alkyl, tri-$C_{1-3}$alkyl)silyl, phenyl, or is as defined under b) or c) in claim 1 or is

d)' a group

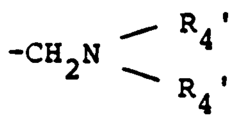

wherein each $R_4$' is allyl or the two $R_4$''s together with the nitrogen atom to which they are attached represent pyrrolidinyl, piperidyl, morpholinyl, thiomorpholinyl with the proviso that when $Ro^{iv}$ is other than chloro, fluoro or methyl it may be only in meta- or para- position or a pharmaceutically acceptable acid addition salt, in the manufacture of a medicament for use in treating tumors.

**4.** The use according to claim 3 whereby in formula Ib R and $R_1$ are as defined in claim 1 and $R_2$''' represents thienyl, furyl or a group

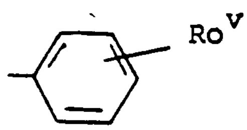

wherein $Ro^{v}$ is in meta or para position and represents $C_{2-6}$alkyl, tri($C_{1-3}$alkyl)silyl, phenyl, is as defined under b) or c) in claim 1 or represents

d)'' a group

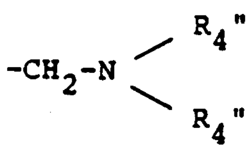

wherein each $R_4$'' is allyl or the two $R_4$'''s together with the nitrogen atom to which they are attached represent pyrrolidinyl or piperidyl.

**5.** A compound of formula Ia

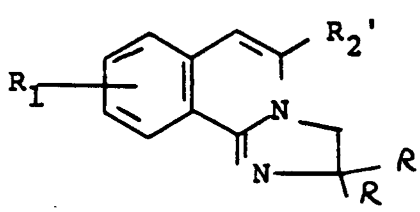

Ia

wherein R and $R_1$ are as defined in claim 1 and

$R_2$' represents thienyl, furyl or a group

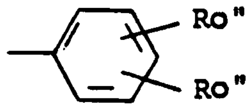

wherein one Ro" is hydrogen and the other represents tri-$C_{1-3}$alkyl)-silyl or phenyl or is as defined under b), c) or d) in claim 1 whereby Ro" substituents may only be in meta or para positions, or a pharmaceutically acceptable acid addition salt thereof.

6. A compound according to claim 5 wherein each R is hydrogen, $R_1$ is in 8-position and is as defined in claim 1 and $R_2$ represents thienyl, furyl or a group

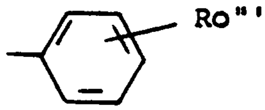

wherein Ro"' is in meta or para position and represents tri-($C_{1-3}$alkyl)silyl or is as defined under b), c) or d) in claim 1.

7. A compound of formula Ia according to claim 5 which is 5[4'-piperidinomethylphenyl]-2,3-dihydroimidazo[2,1-a]isoquinoline; or a pharmaceutically acceptaple addition salt thereof.

8. A compound of formula Ia according to claim 5 which is 5-[4'-(3,4,5-trimethoxyphenylethyl)phenyl]-2,3-dihydroimidazo[2,1-a]-isoquinoline; or a pharmaceutically acceptable acid addition salt thereof.

9. A compound of formula I as defined in claim 1 or of formula Ib as defined in claim 3 which 5-(4'-t-butylphenyl)-2,3-dihydroimidazo[2,1-a]isoquinoline; or a pharmaceutically acceptable acid addition salt thereof.

10. The use according to claim 1 or 3 of a compound according to claim 9

11. A pharmaceutical composition comprising a compound according to any one of claims 5 to 9 together with a pharmaceutically acceptable diluent or carrier.

12. A compound according to any one of claims 5 to 9 for use as a pharmaceutical.

13. A process for preparing a compound fo formula Ia as wherein R and $R_1$ are as defined in claim 1 and $R_2$' is as defined in claim 5 or a pharmaceutically acceptable acid addition salt thereof which comprises dehydrating the corresponding compound of formula V

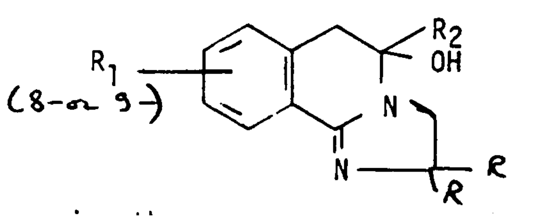

V

wherein R and $R_1$ are as defined in claim 1 and $R_2$ has the significance indicated in claim 5 for $R_2$' and recovering the compound obtained in free form or in the form of a pharmaceutically acceptable acid addition salt.

14. A compound of formula Va

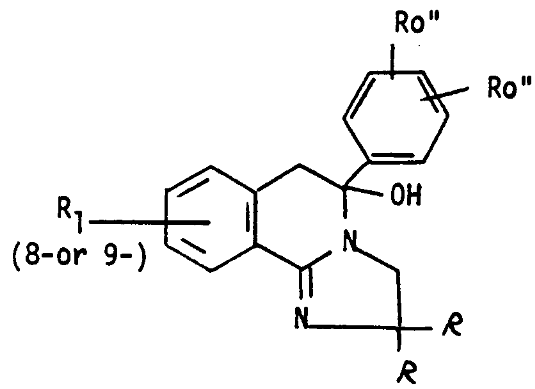

Va

wherein R and $R_1$ are as defined in claim 1 and Ro" is as defined in claim 5.

**Claims for the following Contracting States : ES, GR**

**1.** A process of manufacture of a medicament for use in inhibiting PAF mediated bronchoconstriction and extravasation and PAF mediated endotoxin induced lung injury which comprises mixing a compound of formula I

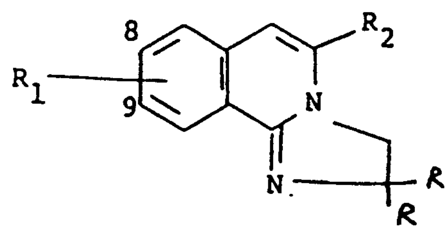

I

wherein each R independently is hydrogen or methyl $R_1$ is in 8- or 9-position and represents hydrogen, chloro, or $C_{1-3}$alkyl and
$R_2$ represents thienyl, furyl or a group

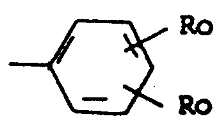

wherein each Ro represents independently hydrogen, fluoro, chloro, $C_{1-10}$alkyl or $C_{1-10}$alkoxy or one Ro is hydrogen and the other represents
a) tri-($C_{1-3}$alkyl)silyl, trifluoromethyl or phenyl;
b) a group

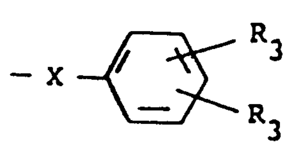

wherein each $R_3$ represents independently hydrogen, chloro, fluoro $C_{1-4}$alkyl or $C_{1-5}$alkoxy and X represents
$-CH_2O-$, $-OCH_2-$, $-CH_2-$, $-CH_2CH_2-$, or $-O-$;
c) a group

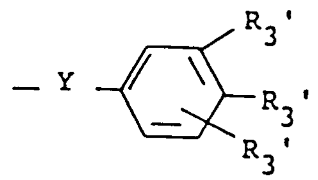

wherein each $R_3'$ represents independently $C_{1-3}$alkoxy and Y represents $-(OCH_2)_{1-5}-$, $-(CH_2)_{1-6}-$ or $-CH_2OCH_2-$ ; or d) a group

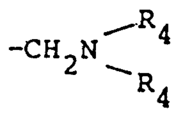

wherein each $R_4$ represents independently $C_{1-4}$alkyl, $C_{5-7}$cycloalkyl, allyl or benzyl; or the two $R_4$'s together with the nitrogen atom to which they are attached represent pyrrolidinyl, piperidyl, morpholinyl or thiomorpholinyl;
or two Ro's on adjacent carbon atoms form methylenedioxy, with the proviso that when an Ro is other than chloro, fluoro, methyl or methoxy it may only be in a meta or para position
or a pharmaceutically acceptable acid addition salt thereof, with a pharmaceutically acceptable carrier or diluent.

2. A process of manufacture of a medicament for use in treating tumors which comprises mixing a compound of formula Ib

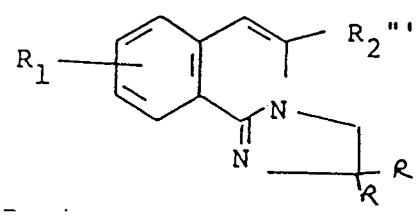

Ib

wherein R and $R_1$ are as defined in claim 1 and
$R_2'''$ represents thienyl, furyl or a group wherein each $Ro^{iv}$ represents independently chloro, fluoro or $C_{1-6}$alkyl or one $Ro^{iv}$ is hydrogen and the other is $C_{2-6}$alkyl, tri-($C_{1-3}$alkyl)silyl, phenyl, or is as defined under b) or c) in claim 1 or is
d)' a group

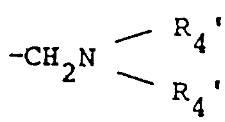

wherein each $R_4'$ is allyl or the two $R_4''$s together with the nitrogen atom to which they are attached represent pyrrolidinyl, piperidyl, morpholinyl or thiomorpholinyl, $Ro^{iv}$ is other than, with the proviso that when $Ro^{iv}$ is other than chloro, fluoro or methyl it may be only in meta- or para- position or a pharmaceutically acceptable acid addition salt thereof, with a pharmaceutically acceptable carrier or diluent.

3. A process of manufacture of a medicament which comprises mixing a compound of formula Ia

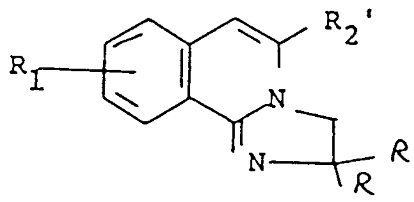

Ia

wherein R and $R_1$ are as defined in claim 1 and $R_2$' represents thienyl, furyl or a group

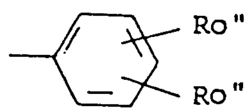

wherein one Ro" is hydrogen and the other represents tri-($C_{1-3}$alkyl)-silyl or phenyl or is as defined under b), c) or d) in claim 1 whereby Ro" substituents may only be in meta or para positions, or a pharmaceutically acceptable acid addition salt thereof, with a pharmaceutically acceptable carrier or diluent.

4. A process for the preparation of a compound of formula Ia as defined in claim 3 or a pharmaceutically acceptable acid addition salt thereof which comprises dehydrating the corresponding compound of formula V wherein R and $R_1$ are as defined in claim 1 and $R_2$ has the significance indicated in claim 3 for $R_2$'

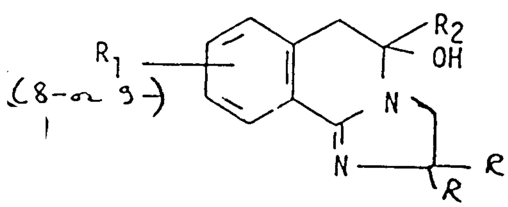

V

wherein R and $R_1$ are as defined in claim 1 and $R_2$ has the significance indicated in claim 3 for $R_2$' and recovering the compound obtained in free form or in the form of a pharmaceutically acceptable acid addition salt.

5. A process according to claim 1, 3 or 4 wherein the compound is 5-[4'-piperidinomethylphenyl]-2,3-dihydroimidazo[2,1-a]isoquinoline or a pharmaceutically acceptable acid addition salt thereof.

6. A process according to claim 1, 3 or 4 wherein the compound is 5-[4'-(3,4,5-trimethoxyphenylethyl)-phenyl]-2,3-dihydroimidazo[2,1-a]-isoquinoline or a pharmaceutically acceptable acid addition salt thereof.

7. A process according to claim 1 or 2 wherein the compound is 5-[4'-t-butylphenyl]-2,3-dihydroimidazo-[2,1-a]isoquinoline or a pharmaceutically acceptable acid addition salt thereof.

**Revendications**

**Revendications pour les Etats contractants suivants : AT, BE, CH, DE, FR, GB, IT, LI, LU, NL, SE**

1. L'utilisation d'un composé de formule I

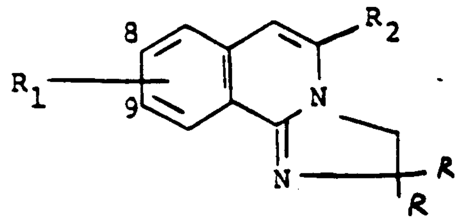

I

dans laquelle

chaque R signifie indépendamment l'hydrogène ou un méthyle,

$R_1$ est en position 8 ou 9 et représente l'hydrogène, le chlore ou un alkyle en $C_1$-$C_3$ et

$R_2$ représente un groupe thiényle, furyle ou un groupe

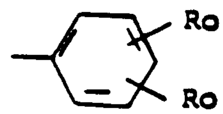

où chaque Ro représente indépendamment l'hydrogène, le fluor, le chlore, un alkyle en $C_1$-$C_{10}$ ou un alcoxy en $C_1$-$C_{10}$, ou bien un Ro signifie l'hydrogène et l'autre représente

a) un groupe tri-(alkyl en $C_1$-$C_3$ )silyle, trifluorométhyle ou phényle,

b) un groupe

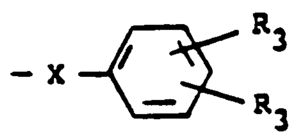

où chaque $R_3$ représente indépendamment l'hydrogène, le chlore, le fluor, un alkyle en $C_1$-$C_4$ ou un alcoxy en $C_1$-$C_5$ et

X représente $-CH_2O-$, $-OCH_2-$, $-CH_2-$, $-CH_2CH_2-$, ou -O-,

c) un groupe

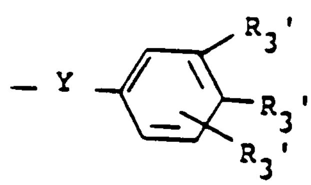

où chaque $R_{3'}$ représente indépendamment un alcoxy en $C_1$-$C_3$ et

Y représente $-(OCH_2)_{1-5}-$, $-(CH_2)_{1-6}-$ ou $-CH_2OCH_2-$ ou

d) un groupe

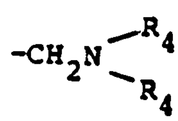

où chaque $R_4$ représente indépendamment un groupe alkyle en $C_1$-$C_4$ , cycloalkyle en $C_5$-$C_7$, allyle ou benzyle, ou bien les deux $R_4$ représentent ensemble, avec l'atome d'azote auquel ils sont fixés, un groupe pyrrolidinyle, pipéridyle, morpholinyle ou thiomorpholinyle;

ou bien les deux Ro sur des atomes de carbone adjacents forment un groupe méthylènedioxy,

avec la condition que lorsque un Ro a une signification autre que le chlore, le fluor, méthyle ou

méthoxy, il ne puisse être qu'en position méta ou para,
ou un sel d'addition d'acide pharmaceutiquement acceptable de ce composé, pour la préparation d'un médicament destiné à inhiber la bronchoconstriction et l'extravasation provoquées par le PAF et les lésions pulmonaires induites par des endotoxines par l'intermédiaire du PAF.

2. L'utilisation selon la revendication 1, caractérisée en ce que, dans la formule I, chaque R signifie l'hydrogène, $R_1$ est tel que défini à la revendication 1 et un Ro signifie l'hydrogène et l'autre est en position méta ou para et est tel que défini sous b) ou c) à la revendication 1.

3. L'utilisation d'un composé de formule Ib

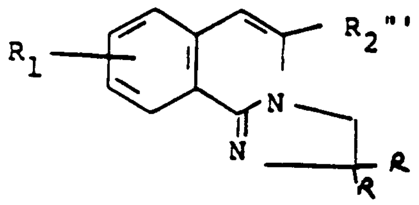

Ib

dans laquelle R et $R_1$ sont tels que définis à la revendication 1 et $R_2'''$ représente un groupe thiényle, furyle ou un groupe

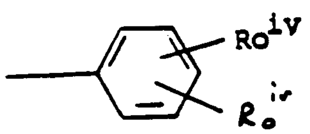

où
chaque $Ro^{iv}$ représente indépendamment le chlore, le fluor ou un alkyle en $C_1$-$C_6$, ou bien un $Ro^{iv}$ signifie l'hydrogène et l'autre est en position méta ou para et représente un groupe alkyle en $C_2$-$C_6$, tri-(alkyl en $C_1$-$C_3$)silyle, phényle ou est tel que défini sous b) ou c) à la revendication 1, ou signifie
d)' un groupe

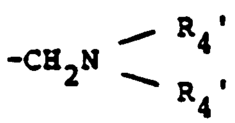

où
chaque $R_4'$ signifie un groupe allyle ou bien les deux $R_4'$ représentent ensemble, avec l'atome d'azote auquel ils sont fixés, un groupe pyrrolidinyle, pipéridyle, morpholinyle, thiomorpholinyle,
avec la condition que lorsque $Ro^{iv}$ a une signification autre que le chlore, le fluor ou méthyle, il ne puisse être qu'en position méta ou para,
ou un sel d'addition pharmaceutiquement acceptable de ce composé, pour la préparation d'un médicament destiné au traitement des tumeurs.

4. L'utilisation selon la revendication 3, caractérisée en ce que, dans la formule Ib, R et $R_1$ sont tels que définis à la revendication 1 et $R_2'''$ représente un groupe thiényle, furyle ou un groupe

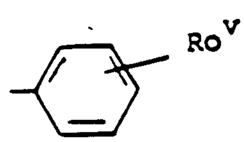

où $Ro^v$ est en position méta ou para et représente un groupe alkyle en $C_2$-$C_6$, tri(alkyl en $C_1$-$C_3$)silyle,

phényle, est tel que défini sous b) ou c) à la revendication 1 ou représente d)" un groupe

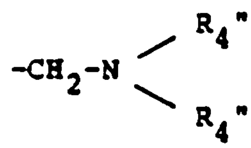

où chaque $R_4$" signifie un groupe allyle ou bien les deux $R_4$" représentent ensemble, avec l'atome d'azote auquel ils sont fixés, un groupe pyrrolidinyle ou pipéridyle.

**5.** Un composé de formule Ia

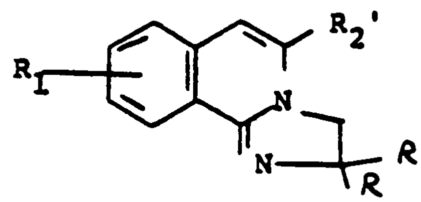

Ia

dans laquelle
R et $R_1$ sont tels que définis à la revendication 1 et
$R_2$' représente un groupe thiényle, furyle ou un groupe

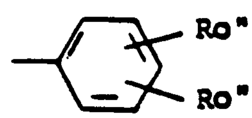

où un Ro" signifie l'hydrogène et l'autre représente un groupe tri-(alkyl en $C_1$-$C_3$)silyle ou phényle ou est tel que défini sous b), c) ou d) à la revendication 1, les substituants Ro" ne pouvant être qu'en position méta ou para, ou un sel d'addition d'acide pharmaceutiquement acceptable de ce composé.

**6.** Un composé selon la revendication 5, dans lequel chaque R signifie l'hydrogène, $R_1$ est en position 8 et est tel que défini à la revendication 1 et $R_2$ représente un groupe thiényle, furyle ou un groupe

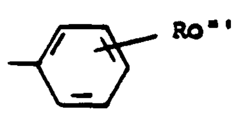

où Ro"' est en position méta ou para et représente un groupe tri(alkyl en $C_1$-$C_3$)silyle ou est tel que défini sous b), c) ou d) à la revendication 1.

**7.** Un composé de formule Ia selon la revendication 5, qui est la 5-[4'-pipéridinométhylphényl]-2,3-dihydro-imidazo[2,1-a]isoquinoléine, ou un sel d'addition d'acide pharmaceutiquement acceptable de ce composé.

**8.** Un composé de formule Ia selon la revendication 5, qui est la 5-[4'-(3,4,5-triméthoxyphényléthyl)-phényl]-2,3-dihydro-imidazo[2,1-a]-isoquinoléine, ou un sel d'addition d'acide pharmaceutiquement acceptable de ce composé.

**9.** Un composé de formule I tel que défini à la revendication 1 ou de formule Ib tel que défini à la revendication 3, qui est la 5-[4'-tert.-butylphényl)-2,3-dihydro-imidazo[2,1-a]isoquinoléine, ou un sel

d'addition d'acide pharmaceutiquement acceptable de ce composé.

**10.** L'utilisation selon la revendication 1 ou 3 d'un composé selon la revendication 9.

**11.** Une composition pharmaceutique, comprenant un composé selon l'une quelconque des revendications 5 à 9 ensemble avec un diluant ou véhicule pharmaceutiquement acceptable.

**12.** Un composé selon l'une quelconque des revendications 5 à 9, pour l'utilisation comme médicament.

**13.** Un procédé de préparation d'un composé de formule Ia où R et $R_1$ sont tels que définis à la revendication 1 et $R_2$' est tel que défini à la revendication 5, ou d'un sel d'addition d'acide pharmaceutiquement acceptable de ce composé, qui comprend la déshydratation du composé correspondant de formule V

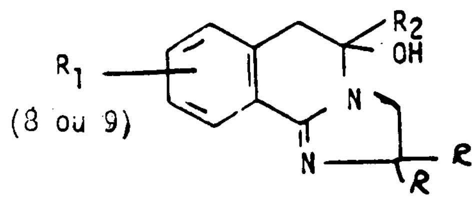

V

dans laquelle R et $R_1$ sont tels que définis à la revendication 1 et $R_2$ a la signification indiquée à la revendication 5 pour $R_2$', et la récupération du composé obtenu sous forme libre ou sous forme d'un sel d'addition d'acide pharmaceutiquement acceptable.

**14.** Un composé de formule Va

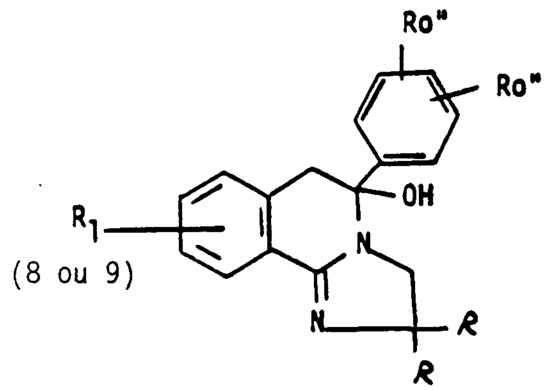

Va

dans laquelle R et $R_1$ sont tels que définis à la revendication 1 et Ro" est tel que défini à la revendication 5.

**Revendications pour les Etats contractants suivants : ES, GR**

**1.** Un procédé de préparation d'un médicament destiné à inhiber la bronchoconstriction et l'extravasation provoquées par le PAF et les lésions pulmonaires induites par des endotoxines par l'intermédiaire du PAF, qui comprend le mélange d'un composé de formule I

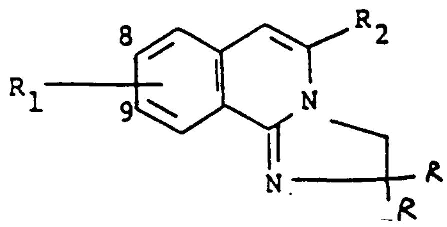

I

dans laquelle

chaque R signifie indépendamment l'hydrogène ou un méthyle,

$R_1$ est en position 8 ou 9 et représente l'hydrogène, le chlore ou un alkyle en $C_1$-$C_3$ et

$R_2$ signifie un groupe thiényle, furyle ou un groupe

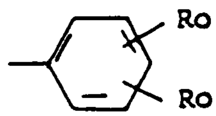

où chaque Ro représente indépendamment l'hydrogène, le fluor, le chlore, un alkyle en $C_1$-$C_{10}$ ou un alcoxy en $C_1$-$C_{10}$, ou bien un Ro signifie l'hydrogène et l'autre représente

a) un groupe tri-(alkyl en $C_1$-$C_3$)silyle, trifluorométhyle ou phényle,

b) un groupe

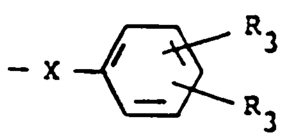

où chaque $R_3$ représente indépendamment l'hydrogène, le chlore, le fluor, un alkyle en $C_1$-$C_4$ ou un alcoxy en $C_1$-$C_5$ et

X représente $-CH_2O-$, $-OCH_2-$, $-CH_2-$, $-CH_2CH_2-$, ou -O-,

c) un groupe

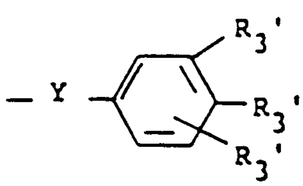

où chaque $R_3$' représente indépendamment un alcoxy en $C_1$-$C_3$ et

Y représente $-(OCH_2)_{1-5}-$, $-(CH_2)_{1-6}-$ ou $-CH_2OCH_2-$ ou

d) un groupe

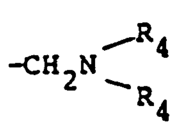

où chaque $R_4$ représente indépendamment un groupe alkyle en $C_1$-$C_4$ , cycloalkyle en $C_5$-$C_7$, allyle ou benzyle, ou bien les deux $R_4$ représentent ensemble, avec l'atome d'azote auquel ils sont fixés,

un groupe pyrrolidinyle, pipéridyle, morpholinyle ou thiomorpholinyle;
ou bien les deux Ro sur des atomes de carbone adjacents forment un groupe méthylènedioxy,
avec la condition que lorsque Ro a une signification autre que le chlore, le fluor, méthyle ou méthoxy, il ne puisse être qu'en position méta ou para,
ou un sel d'addition d'acide pharmaceutiquement acceptable de ce composé,
avec un véhicule ou diluant pharmaceutiquement acceptable.

**2.** Un procédé de préparation d'un médicament pour l'utilisation dans le traitement des tumeurs, qui comprend le mélange d'un composé de formule Ib

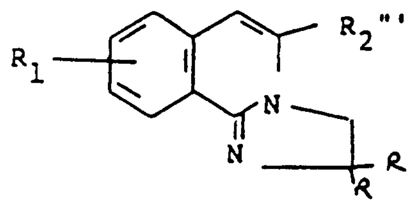

Ib

dans laquelle R et $R_1$ sont tels que définis à la revendication 1 et $R_2'''$ représente un groupe thiényle, furyle ou un groupe

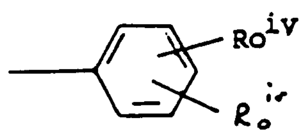

où
chaque $Ro^{iv}$ représente indépendamment le chlore, le fluor ou un alkyle en $C_1$-$C_6$, ou bien un $Ro^{iv}$ signifie l'hydrogène et l'autre signifie un groupe alkyle en $C_2$-$C_6$, tri(alkyl en $C_1$-$C_3$)silyle, phényle, ou est tel que défini sous b) ou c) à la revendication 1 ou signifie
d)' un groupe

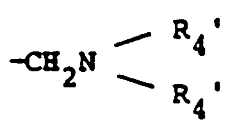

où
chaque $R_4'$ signifie un groupe allyle ou bien les deux $R_4'$ représentent ensemble, avec l'atome d'azote auquel ils sont fixés, un groupe pyrrolidinyle, pipéridyle, morpholinyle ou thiomorpholinyle,
avec la condition que lorsque $Ro^{iv}$ a une signification autre que le chlore, le fluor ou méthyle, il ne puisse être qu'en position méta ou para,
ou un sel d'addition pharmaceutiquement acceptable de ce composé,
avec un véhicule ou diluant pharmaceutiquement acceptable.

**3.** Un procédé de préparation d'un médicament, qui comprend le mélange d'un composé de formule Ia

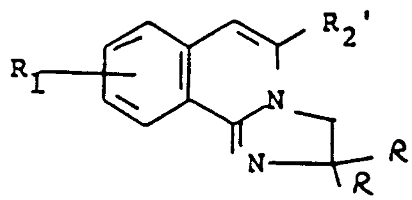

Ia

dans laquelle
R et $R_1$ sont tels que définis à la revendication 1 et $R_2$' représente un groupe thiényle, furyle ou un groupe

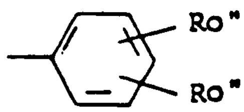

où un Ro" signifie l'hydrogène et l'autre représente un groupe tri-(alkyl en $C_1$-$C_3$)silyle ou phényle ou est tel que défini sous b), c) ou d) à la revendication 1, les substituants Ro" ne pouvant être qu'en position méta ou para,
ou un sel d'addition d'acide pharmaceutiquement acceptable de ce composé,
avec un véhicule ou diluant pharmaceutiquement acceptable.

**4.** Un procédé de préparation d'un composé de formule Ia tel que défini à la revendication 3 ou d'un sel d'addition d'acide pharmaceutiquement acceptable de ce composé, qui comprend la déshydratation du composé correspondant de formule V

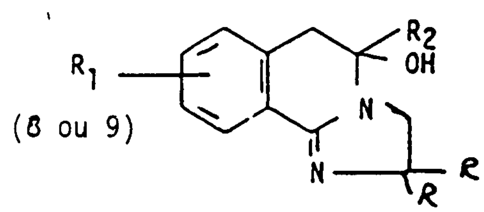

V

dans laquelle R et $R_1$ sont tels que définis à la revendication 1 et $R_2$ a la signification indiquée à la revendication 3 pour $R_2$', et la récupération du composé obtenu sous forme libre ou sous forme d'un sel d'addition d'acide pharmaceutiquement acceptable.

**5.** Un procédé selon la revendication 1, 3 ou 4, dans lequel le composé est la 5-[4'-pipéridinométhylphényl]-2,3-dihydro-imidazo[2,1-a]isoquinoléine, ou un sel d'addition d'acide pharmaceutiquement acceptable de ce composé.

**6.** Un procédé selon la revendication 1, 3 ou 4, dans lequel le composé est la 5-[4'-(3,4,5-triméthoxyphényléthyl)phényl]-2,3-dihydro-imidazo[2,1-a]isoquinoléine, ou un sel d'addition d'acide pharmaceutiquement acceptable de ce composé.

**7.** Un procédé selon la revendication 1 ou 2, dans lequel le composé est la 5-[4'-tert.-butylphényl]-2,3-dihydro-imidazo[2,1-a]isoquinoléine, ou un sel d'addition d'acide pharmaceutiquement acceptable de ce composé.

**Patentansprüche**
**Patentansprüche für folgende Vertragsstaaten : AT, BE, CH, DE, FR, GB, IT, LI, LU, NL, SE**

**1.** Verwendung einer Verbindung der Formel I

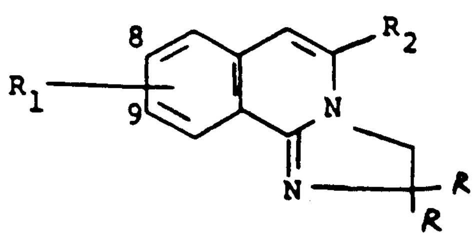

I

worin jeder Rest R unabhängig Wasserstoff oder ein Methylrest ist,
$R_1$ in 8- oder 9-stellung ist und Wasserstoff, Chlor oder einen $C_1$-$C_3$-Alkylrest bedeutet und
$R_2$ einen Thienylrest, Furylrest oder eine Gruppe

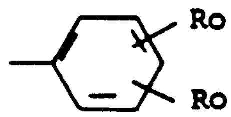

bedeutet, worin jeder Rest Ro unabhängig Wasserstoff, Fluor, Chlor, einen $C_1$-$C_{10}$-Alkyl- oder $C_1$-$C_{10}$-Alkoxyrest bedeutet oder worin ein Rest Ro Wasserstoff ist und der andere

a) einen Tri-($C_1$-$C_3$-alkyl)silyl-, Trifluormethyl- oder Phenylrest bedeutet;

b) eine Gruppe

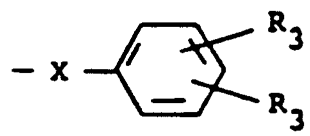

bedeutet, worin jeder Rest $R_3$ unabhängig Wasserstoff, Chlor, Fluor, einen $C_1$-$C_4$-Alkyl- oder $C_1$-$C_5$-Alkoxyrest bedeutet und X $-CH_2O-$, $-OCH_2-$, $-CH_2-$, $-CH_2CH_2-$ oder -O- bedeutet;

c) eine Gruppe

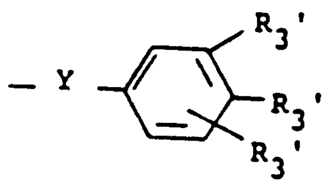

bedeutet, worin jeder Rest $R_3'$ unabhängig einen $C_1$-$C_3$-Alkoxyrest bedeutet und Y $-(OCH_2)_{1-5}-$, $-(CH_2)_{1-6}-$ oder $-CH_2OCH_2-$bedeutet; oder

d) eine Gruppe

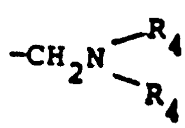

bedeutet, worin jeder Rest $R_4$ unabhängig einen $C_1$-$C_4$-Alkyl-, $C_5$-$C_7$-Cycloalkyl-, Allyl- oder Benzylrest bedeutet oder worin die zwei Reste $R_4$ zusammen mit dem Stickstoffatom, an das sie gebunden sind, eine Pyrrolidinyl-, Piperidyl-, Morpholinyl-oder Thiomorpholinylgruppe darstellen oder worin zwei Reste Ro an benachbarten Kohlenstoffatomen einen Methylendioxyrest bilden, mit dem Vorbehalt, daß dann, wenn ein Rest Ro etwas anderes als Chlor, Fluor, einen Methyl- oder Methoxyrest bedeutet, er nur in meta- oder para-Stellung sein kann,

oder ein pharmazeutisch annehmbares Säureadditionssalz davon zur Herstellung eines Arzneimittels zur Verwendung zur Hemmung von durch PAF vermittelte Bronchienverengerung und Extravasation und durch PAF vermittelte endotoxininduzierte Lungenschadigung.

2. Verwendung nach Anspruch 1, wobei in Formel I jeder Rest R Wasserstoff ist, $R_1$ wie in Anspruch 1 definiert ist und einer der Reste Ro Wasserstoff ist und der andere sich in meta- oder para-Stellung befindet und wie unter b) oder c) in Anspruch 1 definiert ist.

**3.** Verwendung einer Verbindung der Formel Ib

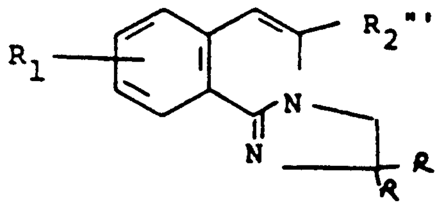

Ib

worin R und $R_1$ wie in Anspruch 1 definiert sind und
$R_2$'" einen Thienylrest, Furylrest oder eine Gruppe

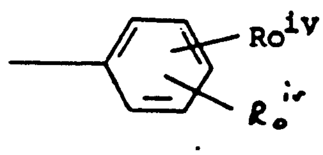

bedeutet, worin jeder Rest $Ro^{iv}$ unabhängig Chlor, Fluor oder einen $C_1$-$C_6$-Alkylrest bedeutet oder einer der Reste $Ro^{iv}$ Wasserstoff ist und der andere sich in meta- oder para-Stellung befindet und einen $C_2$-$C_6$-Alkyl-, Tri-($C_1$-$C_3$-alkyl)silyl- oder Phenylrest bedeutet oder wie unter b) oder c) in Anspruch 1 definiert ist oder

d)'eine Gruppe

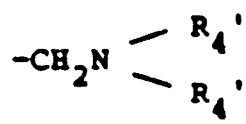

bedeutet, worin jeder Rest $R_4$' ein Allylrest ist oder worin die zwei Reste $R_4$' zusammen mit dem Stickstoffatom, an das sie gebunden sind, eine Pyrrolidinyl-, Piperidyl-, Morpholinyl-, Thiomorpholinylgruppe darstellen, mit dem Vorbehalt, daß dann, wenn $Ro^{iv}$ etwas anderes als Chlor, Fluor oder eine Methylgruppe ist, es nur in meta- oder para-Stellung sein kann,

oder ein pharmazeutisch annehmbares Säureadditionssalz zur Herstellung eines Arzneimittels zur Verwendung zur Behandlung von Tumoren.

**4.** Verwendung nach Anspruch 3, worin in Formel Ib R und $R_1$ wie in Anspruch 1 definiert sind und $R_2$'" einen Thienylrest, Furylrest oder eine Gruppe

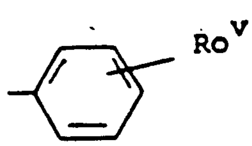

darstellt, worin $Ro^{v}$ in meta- oder para-Stellung ist und einen $C_2$-$C_6$-Alkyl-, Tri-($C_1$-$C_3$-alkyl)silyl-, Phenylrest bedeutet, wie unter b) oder c) in Anspruch 1 definiert ist oder

d)"eine Gruppe

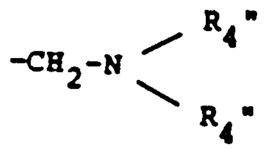

bedeutet, worin jeder Rest $R_4$" ein Allylrest ist oder worin die zwei Reste $R_4$" zusammen mit dem Stickstoffatom, an das sie gebunden sind, eine Pyrrolidinyl- oder Piperidylgruppe darstellen.

**5.** Verbindung der Formel Ia

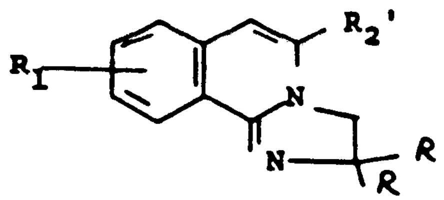

Ia

worin R und $R_1$ wie in Anspruch 1 definiert sind und $R_2$' einen Thienylrest, Furylrest oder eine Gruppe

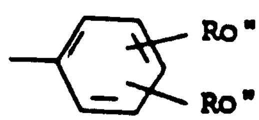

bedeutet, worin ein Rest Ro" Wasserstoff ist und der andere einen Tri-($C_1$-$C_3$-alkyl)silyl- oder Phenylrest bedeutet oder wie unter b), c) oder d) in Anspruch 1 definiert ist, wobei sich die Ro"-Substituenten nur in meta- oder para-Stellung befinden können,
oder ein pharmazeutisch annehmbares Säureadditionssalz davon.

**6.** Verbindung nach Anspruch 5, worin jeder Rest R Wasserstoff ist, $R_1$ sich in 8-Stellung befindet und wie in Anspruch 1 definiert ist und $R_2$ einen Thienylrest, Furylrest oder eine Gruppe

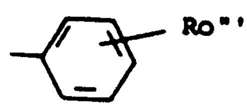

bedeutet, worin Ro'" sich in meta- oder para-Stellung befindet und einen Tri-($C_1$-$C_3$-alkyl)silylrest bedeutet oder wie unter b), c) oder d) in Anspruch 1 definiert ist.

**7.** Verbindung der Formel Ia nach Anspruch 5, die 5-[4'-Piperidinomethylphenyl]-2,3-dihydroimidazo[2,1-a]isochinolin oder ein pharmazeutisch annehmbares Säureadditionssalz davon ist.

**8.** Verbindung der Formel Ia nach Anspruch 5, die 5-[4'-(3,4,5-Trimethoxyphenylethyl)phenyl]-2,3-dihydroimidazo[2,1-a]isochinolin oder ein pharmazeutisch annehmbares Säureadditionssalz davon ist.

**9.** Verbindung der Formel I wie in Anspruch 1 definiert oder der Formel Ib wie in Anspruch 3 definiert, die 5-(4'-t-Butylphenyl)-2,3-dihydroimidazo[2,1-a]isochinolin oder ein pharmazeutisch annehmbares Säureadditionssalz davon ist.

**10.** Verwendung nach Anspruch 1 oder 3 einer Verbindung nach Anspruch 9.

**11.** Pharmazeutische Zusammensetzung enthaltend eine Verbindung nach einem der Ansprüche 5 bis 9 zusammen mit einem pharmazeutisch annehmbaren Verdünnungsmittel oder Träger.

**12.** Verbindung nach einem der Ansprüche 5 bis 9 zur Verwendung als Pharmazeutikum.

**13.** Verfahren zur Herstellung einer Verbindung der Formel Ia, worin R und $R_1$ wie in Anspruch 1 definiert sind und $R_2'$ wie in Anspruch 5 definiert ist oder eines pharmazeutisch annehmbaren Säureadditionssalzes davon, umfassend, daß man die entsprechende Verbindung der Formel V

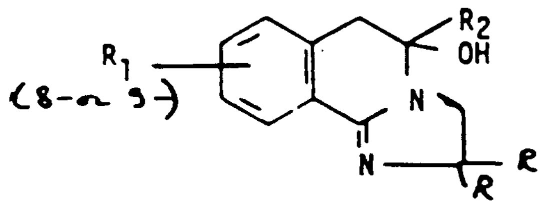

V

worin R und $R_1$ wie in Anspruch 1 definiert sind und $R_2$ die in Anspruch 5 für $R_2'$ angegebene Bedeutung hat, dehydratisiert und die in freier Form oder in Form eines pharmazeutisch annehmbaren Säureadditionssalzes erhaltene Verbindung gewinnt.

**14.** Verbindung der Formel Va

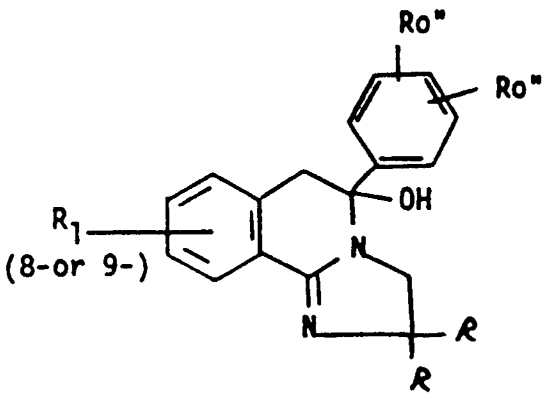

Va

worin R und $R_1$ wie in Anspruch 1 definiert sind und Ro" wie in Anspruch 5 definiert ist.

**Patentansprüche für folgende Vertragsstaaten : ES, GR**

**1.** Verfahren zur Herstellung eines Arzneimittels zur Verwendung zur Hemmung von durch PAF vermittelter Bronchienverengerung und Extravasation und PAF vermittelter endotoxininduzierter Lungenschädigung, umfassend, daß man eine Verbindung der Formel I

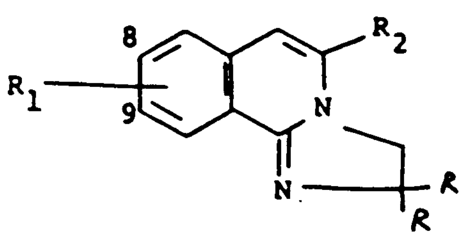

I

worin jeder Rest R unabhängig Wasserstoff oder ein Methylrest ist,
$R_1$ in 8- oder 9-Stellung ist und Wasserstoff, Chlor oder einen $C_1$-$C_3$-Alkylrest bedeutet und
$R_2$ einen Thienylrest, Furylrest oder eine Gruppe

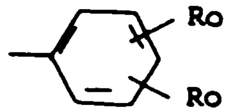

bedeutet, worin jeder Rest Ro unabhängig Wasserstoff, Fluor, Chlor, einen $C_1$-$C_{10}$-Alkyl- oder $C_1$-$C_{10}$-Alkoxyrest bedeutet oder worin ein Rest Ro Wasserstoff ist und der andere

a) einen Tri-($C_1$-$C_3$-alkyl)silyl-, Trifluormethyl- oder Phenylrest bedeutet;

b) eine Gruppe

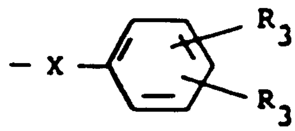

bedeutet, worin jeder Rest $R_3$ unabhängig Wasserstoff, Chlor, Fluor, einen $C_1$-$C_4$-Alkyl- oder $C_1$-$C_5$-Alkoxyrest bedeutet und X $-CH_2O-$, $-OCH_2-$, $-CH_2-$, $-CH_2CH_2-$ oder -O- bedeutet;

c) eine Gruppe

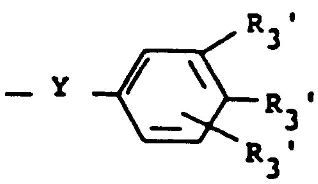

bedeutet, worin jeder Rest $R_3'$ unabhängig einen $C_1$-$C_3$-Alkoxyrest bedeutet und Y $-(OCH_2)_{1-5}-$, $-(CH_2)_{1-6}-$ oder $-CH_2OCH_2-$ bedeutet; oder

d) eine Gruppe

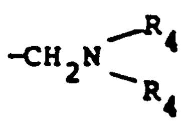

bedeutet, worin jeder Rest $R_4$ unabhängig einen $C_1$-$C_4$-Alkyl-, $C_5$-$C_7$-Cycloalkyl-, Allyl- oder Benzylrest bedeutet oder worin die zwei Reste $R_4$ zusammen mit dem Stickstoffatom, an das sie gebunden sind, eine Pyrrolidinyl-, Piperidyl-, Morpholinyl- oder Thiomorpholinylgruppe darstellen oder worin zwei Reste Ro an benachbarten Kohlenstoffatomen einen Methylendioxyrest bilden, mit dem Vorbehalt, daß dann, wenn ein Rest Ro etwas anderes als Chlor, Fluor, einen Methyl- oder Methoxyrest bedeutet, er nur in meta- oder para-Stellung sein kann,

oder ein pharmazeutisch annehmbares Säureadditionssalz davon mit einem pharmazeutisch annehmbaren Träger oder Verdünnungsmittel vermischt.

**2.** Verfahren zur Herstellung eines Arzneimittels zur Verwendung zur Behandlung von Tumoren, umfassend, daß man eine Verbindung der Formel Ib

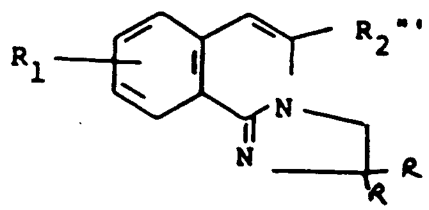

Ib

worin R und $R_1$ wie in Anspruch 1 definiert sind und $R_2'''$ einen Thienylrest, Furylrest oder eine Gruppe

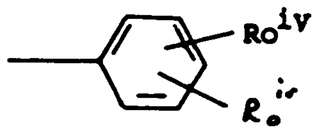

bedeutet, worin jeder Rest $Ro^{iv}$ unabhängig Chlor, Fluor oder einen $C_1$-$C_6$-Alkylrest bedeutet oder einer der Reste $Ro^{iv}$ Wasserstoff ist und der andere einen $C_2$-$C_6$-Alkyl-, Tri-($C_1$-$C_3$-alkyl)silyl- oder Phenylrest bedeutet oder wie unter b) oder c) in Anspruch 1 definiert ist oder

d)'eine Gruppe

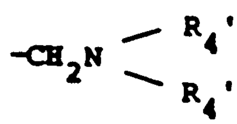

bedeutet, worin jeder Rest $R_4'$ ein Allylrest ist oder worin die zwei Reste $R_4'$ zusammen mit dem Stickstoffatom, an das sie gebunden sind, eine Pyrrolidinyl-, Piperidyl-, Morpholinyl- oder Thiomorpholinylgruppe darstellen, mit dem Vorbehalt, daß dann, wenn $Ro^{iv}$ etwas anderes als Chlor, Fluor oder eine Methylgruppe ist, es nur in meta- oder para-Stellung sein kann,

oder ein pharmazeutisch annehmbares Säureadditionssalz davon mit einem pharmazeutisch annehmbaren Träger oder Verdünnungsmittel vermischt.

**3.** Verfahren zur Herstellung eines Arzneimittels, umfassend, daß man eine Verbindung der Formel Ia

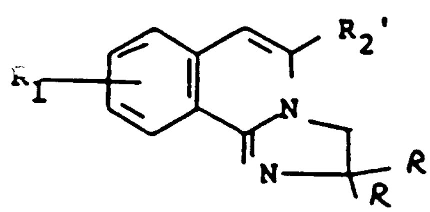

Ia

worin R und $R_1$ wie in Anspruch 1 definiert sind und $R_2'$ einen Thienylrest, Furylrest oder eine Gruppe

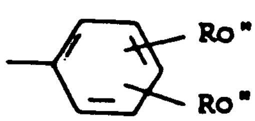

bedeutet, worin ein Rest Ro" Wasserstoff ist und der andere einen Tri-($C_1$-$C_3$-alkyl)silyl- oder Phenylrest bedeutet oder wie unter b), c) oder d) in Anspruch 1 definiert ist, wobei die Ro"-Substituenten nur in meta- oder para-Stellung sein können, oder ein pharmazeutisch annehmbares Säureadditionssalz davon mit einem pharmazeutisch annehmbaren Träger oder Verdünnungsmittel vermischt.

**4.** Verfahren zur Herstellung einer Verbindung der Formel Ia wie in Anspruch 3 definiert oder eines pharmazeutisch annehmbaren Säureadditionssalzes davon, umfassend, daß man die entsprechende Verbindung der Formel V

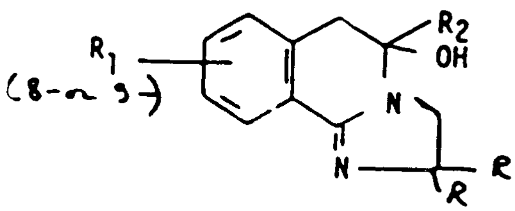

V

worin R und $R_1$ wie in Anspruch 1 definiert sind und $R_2$ die in Anspruch 3 die für $R_2$' angegebene Bedeutung hat, dehydratisiert und die in freier Form oder in Form eines pharmazeutisch annehmbaren Säureadditionssalzes erhaltene Verbindung gewinnt.

**5.** Verfahren nach Anspruch 1, 3 oder 4, worin die Verbindung 5-[4'-Piperidinomethylphenyl]-2,3-dihydroimidazo[2,1-a]isochinolin oder ein pharmazeutisch annehmbares Säureadditionssalz davon ist.

**6.** Verfahren nach Anspruch 1, 3 oder 4, worin die Verbindung 5-[4'-(3,4,5-Trimethoxyphenylethyl)phenyl]-2,3-dihydroimidazo[2,1-a]isochinolin oder ein pharmazeutisch annehmbares Säureadditionssalz davon ist.

**7.** Verfahren nach Anspruch 1 oder 2, worin die Verbindung 5-[4'-t-Butylphenyl]-2,3-dihydroimidazo[2,1-a]-isochinolin oder ein pharmazeutisch annehmbares Säureadditionssalz davon ist.